# EUROPEAN PATENT APPLICATION

(11) **EP 2 384 743 A1**
(43) Date of publication of application: **09.11.2011**
(21) Application number: 10075170.0
(22) Date of filing: 27.04.2010
(51) Int. Cl.: A61K 9/127, A61K 31/704, A61K 9/00

(54) **Siosomal formulation for intracellular delivery and targeting of therapeutic agents**

(71) Applicant: Salama, Zoser B., 88214 Ravensburg (DE)
(72) Inventor: Salama, Zoser B., 88214 Ravensburg (DE)
(74) Representative: Boeckh, Tobias

(57) **Abstract**

The invention relates to a carrier and targeting system for the intracellular delivery and triggered release of a siosome- and/or siosome-liposome-entrapped or encapsulated active substance or therapeutic agent. The invention further relates to the targeted release and delivery of doxorubicin, cis-platin, carbo-platin and/or oxali-platin, characterized in that one or more anionic lipids, such as cholesteryl hemisuccinate residues, cardiolipin and/or their derivatives and/or other anionic groups, are covalently linked to the silicone central atom of the silanes, sugar silanes and/or amino-sugar silanes. The invention further relates to the use of cationic lipids that are covalently attached to the silicone central atom of the silanes. The invention also relates to a method for preparing such carrier and targeting systems and methods for their administration.

## Description

### Field of Invention

The invention relates to a carrier and targeting system for the intracellular delivery and triggered release of a siosome- and/or siosome-liposome-entrapped or encapsulated active substance or therapeutic agent. The invention further relates to the targeted release and delivery of doxorubicin, cis-platin, carbo-platin and/or oxali-platin, characterized in that one or more anionic lipids, such as cholesteryl hemisuccinate residues, cardiolipin and/or their derivatives and/or other anionic groups, are covalently linked to the silicone central atom of the silanes, sugar silanes and/or amino-sugar silanes. The invention further relates to the use of cationic lipids that are covalently attached to the silicone central atom of the silanes. The invention also relates to a method for preparing such carrier and targeting systems and methods for their administration.

### Background of the invention

It is well recognised in the medical field that the most effective procedures for treating localised diseases relates to directing a pharmaceutical agent or active substance to the affected area, thereby avoiding undesirable toxic effects of systemic treatment. Techniques and methods currently used to deliver active substances to specific target sites within the body involve the utilisation of time-release delivery systems.

Typically, the encapsulation of an active substance in a vesicle can alter the pattern of bio-distribution and the pharmacokinitecs of the active substance. In certain cases such as liposomal encapsulation, it has been found that toxicity can be lowered. In particular, the so called "long circulating liposomal formulations", which avoid uptake by the organs of the mononuclear phagocyte system, primarily in the liver and spleen, have been extensively studied.

Such long circulating liposomes may include a surface coat of flexible water soluble chains that act to prevent interaction between the liposome and plasma components which play a role in liposome uptake. Otherwise, such liposomes can be produced without this coating, whereby saturated long chain phospholipid and cholesterol compounds are used instead.

Doxorubicin (DXR) is a potent chemoactive substance effective against a broad spectrum of neoplasms. However, clinical use of the drug in free form or as doxorubicin hydrochloride liposomal, Doxil ® (formulated in Stealth ® liposomes composed of N-(carbomoyl-methoxypolyethylene glycol 2000)-1,2-distearoyl-sn-glycero-3-phosphoethanolamine sodium [MPEG-DSPE], hydrogenated soy phosphatidyl-choline [HSPC], and cholesterol) is limited by serious side effects and acute toxicity including malaise, nausea, vomiting, myelosuppression, and severe alopecia. In addition, cumulative and irreversible cardiac damage occurs after repeated administration, which seriously limits the use of the drug, although the DXR in liposomes achieved increased therapeutic action and reduced toxicity.

The degradation of the lipid and active substances which can occur on storage will have an important impact on the efficacy of the formulation. Phospholipid degradation in liposomes for example can take the form of hydrolytic release of fatty acyl chain groups and lipid peroxidative damage, particularly at unsaturated bound regions and lipid acyl chain moieties. In addition, drugs such as DXR are themselves capable of initiating oxidative reactions, and in the presence of lipids, appear to contribute to free radical/oxidative reactions, and also undergo rapid chemical changes on storage in liposomes.

The lipid concentration in the DXR/liposomes intended for intravenous administration in cancer chemotherapy is very high, of at least 100 mM liposome lipid in addition to other additives as cryoprotectants (1-10%).

The exact mechanism of action of doxorubicin is complex and still somewhat unclear, although it is to interact with DNA by intercalation. Doxorubicin is known to interact with DNA by intercalation and inhibition of macromolecular biosynthesis. This inhibits the progression of the enzyme topoisomerase II, which unwinds the DNA for transcription.

Doxorubicin stabilises the topoisomerase II complex after it has broken the DNA chain for replication, preventing the DNA double helix from being resealed and thereby stopping the process of replication. The planar aromatic chromophone position of the molecule intercalates between two base pairs of the DNA, while the six-membered sugar sits in the minor groove and interacts with flanking base pairs immediately adjacent to the intercalation site, as evidenced by several crystal structure studies. Doxorubicin cardiotoxicity is characterized by a dose-dependent decline in mitochondrial oxidative phosphorylation. Reactive oxygen species, which are generated by the interaction of doxorubicin with iron, can then damage the myocytes (heart cells), causing myofibrillar loss and cytoplasmic vacuolisation.

The drug is administered intravenously in the form of hydrochloride salt, as a lysosomal doxorubicin for injection and PEGylated lysosomal doxorubicin (Doxil®). All formulations of doxorubicin hydrochloride have shown serious adverse effects. A summary of the adverse effects is provided below:

### 1. Hematologic effects

- Leukopenia (principally granulocytopenia) is the predominant manifestation of hematologic toxicity, the severity of which depends on the dose of the drug and on the regenerative capacity of the bone marrow.
- The principal dose-limiting toxicity of PEG-stabilised liposomal doxorubicin in patients with AIDS-related Kaposi's sarcoma has been myelosuppression, commonly manifested by leukopenia and neutropenia; anaemia and thrombocytopenia also occur frequently.

### 2. Cardiac effects

These include congestive heart failure, dilated cardiomyopathy, and death. Doxorubicin cardiotoxicity is characterized by a dose-dependent decline in mitochondrial oxidative phosphorylation. Reactive oxygen species, generated by the interaction of doxorubicin with iron, can then damage the myocytes (heart cells), causing myofibrillar loss and cytoplasmic vacuolisation.

### 3. Gastro-intestinal effects (GI effects)

Stomatitis and esophagitis (mucositis) may occur in patients receiving doxorubicin. GI toxicity (evidenced frequently by nausea and vomiting and occasionally by anorexia and diarrhoea) may occur, usually on the day of drug administration.

### 4. Dermatologic effects

Complete alopecia almost always accompanies doxorubicin therapy.

### 5. Nervous system effects

Peripheral neurotoxicity.

Small interfering RNA (siRNA), also called silencing RNA or short interfering RNA, was first implicated in post-transcriptional gene silencing (PTGS) in plants (Hamilton et al., 1999).

The siRNA which contains 21-25 base pairs, plays an important role in silencing an RNA pathway. It describes the specific knock-down of target gene (target sequences) of interest by an artificial mechanism. The siRNA delivery (or transfection) reagent complexes are often specifically referred to as lipoplexes, dedriplexes and polyplexes, depending on whether the vector used is a cationic lipid, dendrimer, or polymer, respectively. Alternatively, these cationic lipids and polymers can form nanoparticles, in which the nucleic acid is entrapped within the body of the particle and not simply adsorbed onto the surface, usually via ionic interaction, as is the case with complexes. However, in the literature, the term nanoparticle is often interchangeably used for complexes, possibly to better reflect the nomenclature being used within the ongoing nanotechnology/nanomedicine field.

Although the exact mechanism of delivery varies, these complexes and nanoparticles can, to varying degrees, provide siRNA stability within blood serum, avoid sequestration within the RES, and eventually reach target cells.

They are taken into the cell by some form of vesicular transport (endocytosis and/or macropinocytosis). Complexes and nanoparticles generally have to be less than 100 nm to avoid renal excretion and be taken up by the target cells.

The encapsulation, entrapment efficiency and the intracellular delivery and targeting of siRNA, DNA, RNA and other nucleosides are very low and limited for a variety of the cited reasons, such as that vesicles, after systemic administration to the blood stream, are rapidly removed from circulation by the reticulo-endothelial system. Another reason is the inherent difficulty in delivering a molecule, in particular a large or a charged molecule, into the cellular cytoplasm and/or the nucleus.

It has been difficult to deliver the gene to the required areas of the body safely and in sufficient quantities, especially in a form stable enough for good expression of the protein for which it codes.

Viruses have been used as delivery vectors but have fallen out of favour somewhat because of toxicity issues, and attention has turned to non-viral alternatives. Positively charged (cationic) synthetic molecules will readily bind to negatively charged DNA molecules and have been used for DNA delivery, but these cationic molecules are often toxic to cells. Naturally occurring negatively charged lipids (anionic) are used as an alternative that do not harm cells.

In view of the foregoing, there exists a need in the art for a method for targeted intracellular drug delivery that overcomes the disadvantages of the currently available methods. Specifically, a delivery systems is required that is stable in the circulation, following intravenous administration, allowing retention of encapsulated or associated drug or active substances. This delivery system would be capable of accumulating at a target organ, tissue or cell via either active targeting (for example by incorporating an antibody or saccharide, nucleoside, peptide, hormone, ligand on the surface of the siosomal vesicle).

Following accumulation at the target site, the siosomal carrier would become capable of fusing without the need for any external stimulus, and would subsequently release any encapsulated or associated drug or active substance in the vicinity of the target cell, or fuse with the target cell plasma membrane, introducing the drug or active substance into the cell cytoplasm. In certain instances, fusion of the carrier with the plasma membrane would be preferred because this would provide more specific drug delivery and hence minimise any adverse effects on normal healthy cells or tissues.

In addition, in the case of active substances such as DNA, RNA, siRNA, proteins, peptides etc., which are generally not permeable to the cell membrane, such a fusogenic carrier would provide a mechanism whereby the active substance could be delivered to its required intracellular site of action.

Further, by avoiding the endocytic pathway, the active substance would not be exposed to acidic conditions and/or degradative enzymes that could be destructive to the said active substance. Quite surprisingly, the present invention addresses this need by providing such a method.

### SUMMARY OF THE INVENTION

The technical problem underlying the present invention is to provide a novel carrier and targeting system for the intracellular delivery and triggered release of a siosome and/or siosome-liposome entrapped agent/active substance from the enzyme-rich acidic environment of the lysosomes, that overcomes the disadvantages of the prior art.

This problem is solved by the features of the independent claims.

Preferred embodiments of the present invention are provided by the dependent claims.

The object of the invention is therefore to provide a carrier and targeting system for the intracellular delivery and release of a siosome- and/or siosome-liposome-entrapped active substance, comprising organosilicon, sugar organosilicon, amino-sugar organosilicon, alkyl, alkoxy or acyloxy organosilicon, peptide organosilicon, amino acid(s) organosilicon, lipids and/or their derivatives.

In a preferred embodiment the carrier and targeting system of the present invention is characterized in that the organosilicon, sugar organosilicon, or amino-sugar organosilicon compounds are characterized by the general formula (I): 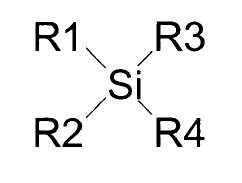 whereby R₁ and R₂ can be the same or different, and where R₃ and R₄ can be the same or different, whereby
- R₁, R₂ =: Mono, Di-, oligosaccharide, nucleotide, alkyl or aryl, aromatic or aliphatic heterocycles, and
- R₃, R₄ =: Polynucleotide, peptide, aliphatic chains, proteins, siRNA, RNA, DNA, ligand, vector, fatty acid, acyl group.

In a further preferred embodiment the carrier and targeting system of the present invention is characterized in that one or more cationic lipids are covalently attached to the silicon and/or as a derivative of the chemical groups and/or residues of R_{1,} R₂, R₃ and R₄ of the general formula (I), whereby
- R₁ =: Monosaccharide, di-saccharide, oligosaccharide, derivatives;
- R₂ =: Monosaccharide, di-saccharide, oligosaccharide, amino acid, peptide, nucleoside, nucleotides, alkyl, aryl residues, aromatic or aliphatic heterocycles;
- R₃, R₄ =: 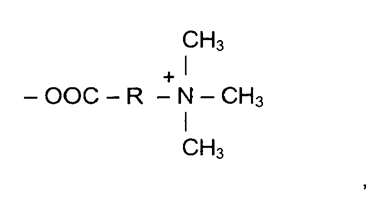 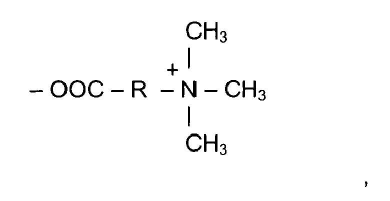 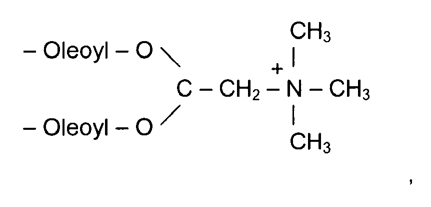
or a peptide residue of the formula: 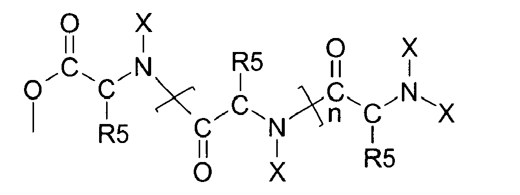 wherein R represents an unbranched or branched alkyl, alkenyl or alkinyl residue with 5 to 29 C atoms which can be substituted by one to three halogen atoms, alkoxy residues with 1 to 18 C atoms or amino groups, the residues R5, which can be the same or different, represent the residue remaining after the removal of the group 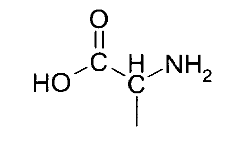 from an amino acid, the residues X, which may be the same or different, represent a hydrogen atom or an amino-protective group usually occurring in peptide chemistry and n represents an integer from 1 to 12 and R₃ and R₄ which can be the same or different, each represent an aryl group, such as a phenyl group, or the residue remaining after the removal of a hydrogen atom from a monosaccharide, disaccharide, amino sugar or a hydroxyl carbon acid, or alkoxy residues with 1-5 C atoms, or acyl residues of an amino acid with a free or protected amino group, or a dipeptide, tripeptide or tetrapeptide residue of an amino acid with free or protected amino groups, or they have the meaning R₁ and R₂, with R representing an unbranched or branched alkyl, akenyl or alkinyl residue.

In a further preferred embodiment the carrier and targeting system of the present invention is characterized in that one or more anionic lipids, such as cholesteryl hemisuccinate residues, cardiolipin and/or their derivatives and/or other anionic groups, are covalently linked to the silicone central atom of the silanes, sugar silanes and/or amino-sugar silanes and/or to one or more of the groups R₁, R₂, R₃ and R₄ of the general formula (I), whereby
- R₁ =: Monosaccharide, di-saccharide, oligosaccharide, derivatives;
- R₂ =: Monosaccharide, di-saccharide, oligosaccharide, amino acid, peptide, nucleoside, nucleotides, alkyl, aryl residues, aromatic or aliphatic heterocycles;
- R₃, R₄ =: Anionic Lipid, cholesteryl hemisuccinate residue, cardiolipin residue, phosphatidylglycerol residue,
or a peptide residue of the formula: 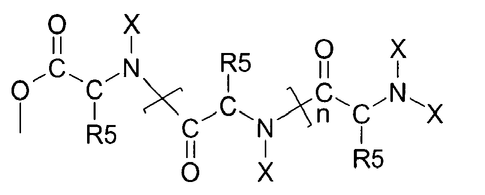 wherein R represents an unbranched or branched alkyl, alkenyl or alkinyl residue with 5 to 29 C atoms which can be substituted by one to three halogen atoms, alkoxy residues with 1 to 18 C atoms or amino groups, the residues R5, which can be the same or different, represent the residue remaining after the removal of the group 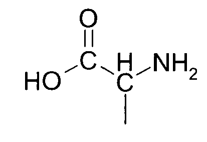 from an amino acid, the residues X, which may be the same or different, represent a hydrogen atom or an amino-protective group usually occurring in peptide chemistry and n represents an integer from 1 to 12 and R₃ and R₄ which can be the same or different, each represent an aryl group, such as a phenyl group, or the residue remaining after the removal of a hydrogen atom from a monosaccharide, disaccharide, amino sugar or a hydroxyl carbon acid, or alkoxy residues with 1-5 C atoms, or acyl residues of an amino acid with a free or protected amino group, or a dipeptide, tripeptide or tetrapeptide residue of an amino acid with free or protected amino groups, or they have the meaning R₁ and R₂, with R representing an unbranched or branched alkyl, akenyl or alkinyl residue.

In a further preferred embodiment the carrier and targeting system of the present invention is characterized in that the encapsulated and/or entrapped active substance is an anti-cancer agent, such as doxorubicin, cis-platin, carbo-platin and/or oxali-platin.

In a further preferred embodiment the carrier and targeting system of the present invention is characterized in that the encapsulated and/or entrapped active substance is genetic material, such as DNA, RNA, siRNA and/or an anti-viral and/or anti-bacterial agent.

In a further preferred embodiment the carrier and targeting system of the present invention is characterized in that polyethylene-glycol is covalently attached to the silicone and/or one or more of the groups R₁, R₂, R₃ and R₄ of the general formula (I).

In a further preferred embodiment the carrier and targeting system of the present invention is characterized in that the covalent attachment of polyethylene-glycol can be carried out by means of a cleavable spacer linker.

In a further preferred embodiment the carrier and targeting system of the present invention is characterized in that the composition is lyophilized.

In a further preferred embodiment the carrier and targeting system of the present invention is characterized in that the composition comprises a cryoprotectant.

In a further preferred embodiment the carrier and targeting system of the present invention is characterized in that the cryoprotectant is a disaccharide selected from the group consisting of sucrose, maltose, trehalose, and/or lactose.

In a further preferred embodiment the carrier and targeting system of the present invention is characterized in that the cryoprotectant is a disaccharide having a concentration of 1-40%.

In a further preferred embodiment the carrier and targeting system of the present invention comprises additionally of liposomes, unsaturated lipids, a vesicle-forming lipid, a hydrophilic polymer and/or liposomes.

In a further preferred embodiment the carrier and targeting system of the present invention is characterized in that the vesicle-forming lipids exhibit two hydrocarbon chains, typically acyl chains, and a polar head group.

In a further preferred embodiment the carrier and targeting system of the present invention is characterized in that the phospholipids are selected from the group comprising phosphatidylcholine (PC), phosphatidylethanolamine (PE), phosphatidic acid (PA), Phosphatidyl-inositol (PI), and sphingomyelin (SM).

In a further preferred embodiment the carrier and targeting system of the present invention is characterized in that the two hydrocarbon chains are between 8-26 carbon atoms in length.

In a further preferred embodiment the carrier and targeting system of the present invention, it is intended that the carrier system comprises additionally of glycolipids such as cerebiosides and gangliosides.

In a further preferred embodiment the carrier and targeting system of the present invention is characterized in that the carrier and targeting system is obtainable by:
a) mixing one or more organosilicon, sugar organosilicon, amino-sugar organosilicon, alkyl, alkoxy or acyloxy organosilicon, peptide organosilicon, amino acid(s) organosilicon, lipids and/or their derivatives, salts and/or the vesicles formed from them, with one or more polymers, such as PEG, one or more salts, such as Zn²⁺, Ca²⁺, Mg²⁺, Na⁺, buffers, with one or more active substances, such as doxorubicin, cis-oxoplatin, oxali-platin, nucleic acids, such as DNA, RNA, siRNA, nucleosides at selected pH, salt concentration and temperature;
b) homogenisation, sonication and/or extrusion of the mixture, followed by
c) separation of the free active substance,
d) sterile filtration of the mixture,
e) lyophilisation,
f) reconstitution to form a siosome and/or a combined siosome-liposome complex concentrate.

A further aspect of the invention is a method for preparing the carrier and targeting system of the present invention, characterized by
g) mixing one or more organosilicon, sugar organosilicon, amino-sugar organosilicon, alkyl, alkoxy or acyloxy organosilicon, peptide organosilicon, amino acid(s) organosilicon, lipids and/or their derivatives, salts and/or the vesicles formed from them, with one or more polymers, such as PEG, one or more salts, such as Zn²⁺, Ca²⁺, Mag²⁺, Na⁺, buffers, with one or more active substances, such as doxorubicin, cis-oxoplatin, oxali-platin, nucleic acids, such as DNA, RNA, siRNA, nucleosides at selected pH, salt concentration and temperature;
h) homogenisation, sonication and/or extrusion of the mixture, followed by
i) separation of the free active substance,
j) sterile filtration of the mixture,
k) lyophilisation,
I) reconstitution to form a siosome and/or a combined siosome-liposome complex concentrate.

In a further preferred embodiment of the present invention the method for producing the carrier and targeting system is characterized in that the active substance, preferably nucleic acids, DNA, RNA and/or siRNA, can be loaded with cations prior to preparation of the siosome carrier systems.

In a further preferred embodiment of the present invention the method for preparing the carrier and targeting system is characterized in that the cationic silanes and/or siosomes can be loaded with anionic salts.

In a further preferred embodiment of the present invention the method for preparing the carrier and targeting system is characterized in that the anionic silanes and/or siosomes can be loaded with cationic salts.

In a further preferred embodiment of the present invention the method for preparing the carrier and targeting system is characterized in that the mixing is carried out in a buffer solution that comprises TRIS, HEPES; MOPS, MES and/or other commonly used biological buffers.

In a further preferred embodiment of the present invention the method for preparing the carrier and targeting system is characterized in that the method includes one or more additives selected from the group comprising of detergent solution, adjuvant solution, salt solution, PEG-conjugates and/or cryoprotectants.

A further aspect of the present invention relates to a method for administering the carrier and targeting system, siosome- and/or siosome-liposome-complex via oral, rectal, vaginal, topical, nasal, intradermal, or parenteral administration.

In a preferred embodiment the method for administering the carrier and targeting system is characterized in that the parenteral administration method is selected from the group comprising subcutaneous, intravenous, intramuscular and infusion.

A further aspect of the present invention relates to a method for treating solid and haematological malignancies in animals and humans comprising the administration of the carrier and targeting system of the present invention.

A further aspect of the present invention relates to a method for increasing intracellular concentration of a siosome-entrapped active substance, preferably doxorubicin, cis-platin and/or siRNA, characterized by administering the carrier and targeting system of the present invention to a patient or a target cell.

In a preferred embodiment the method for increasing intracellular concentration of a siosome-entrapped active substance is characterized in that the intracellular concentration of the active substance is increased by specific binding of a ligand to a target cell and/or internalisation of the siosome- and/or siosome-liposome-complex by the target cell. In a further preferred embodiment the method is characterized in that the intracellular cytotoxicity of a tumor cell or other target cell is increased by administration of the carrier and targeting system according to the present invention. The present invention therefore provides siosomal compositions, carrier systems and methods using systems for intracellular delivery and triggered release by siosome-entrapped agents or active substances from the enzyme-rich acidic environment of the lysosomes (pH-sensitive, triggered release siosomes).

A further intended aspect of the present invention is the use of the carrier and targeting system as described herein as a medicament.

A further aspect of the present invention relates to a pharmaceutical agent comprising the carrier and targeting system as described herein and a pharmaceutically acceptable carrier.

A further aspect of the invention also relates to a method of transfecting a cell with an anti-cancer active substance or therapeutic agent such as doxorubicin, cis-platin, carbo-platin, oxali-platin, bleomycin, a nucleic acid, such as DNA, RNA, siRNA, an anti-viral active substance and/or an anti-bacterial agent, using as the transfection vector a carrier and targeting system according to the present invention comprising at least one sugar organosilicon compound and/or derivatives thereof as described herein, preferably in addition to PEG and/or a cryoprotectant.

The procedures according to the present application produce a carrier system complex that is unexpectedly stable in comparison to the prior art siosome encapsulation, entrapment and/or complex formation, that is, the carrier system of the present invention is easier to produce, has a higher encapsulation efficiency and exhibits a higher intracellular concentration of active agent, and leads to more specific cell targeting of the active substances and also exhibits less toxicity.

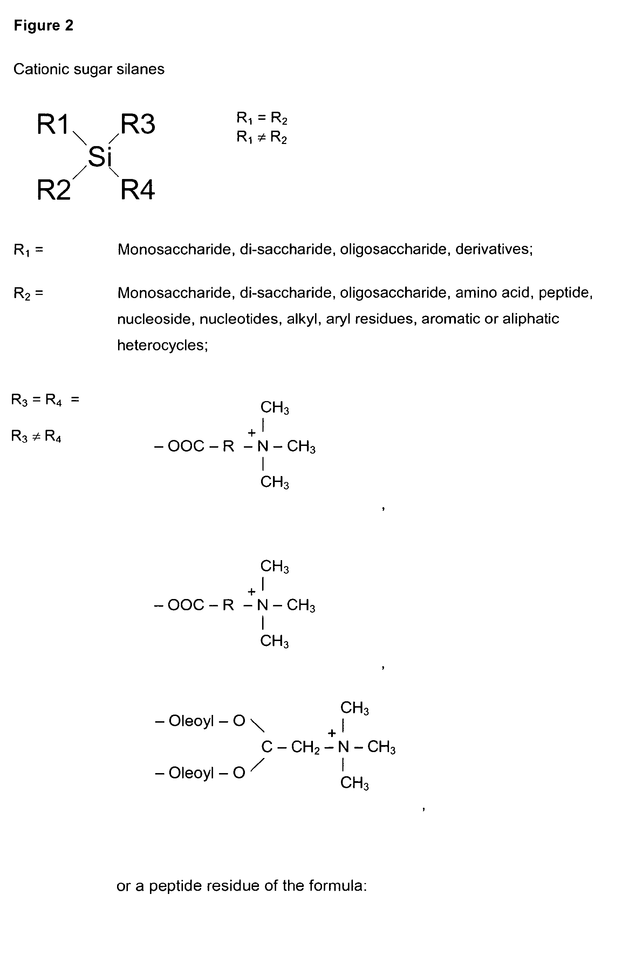

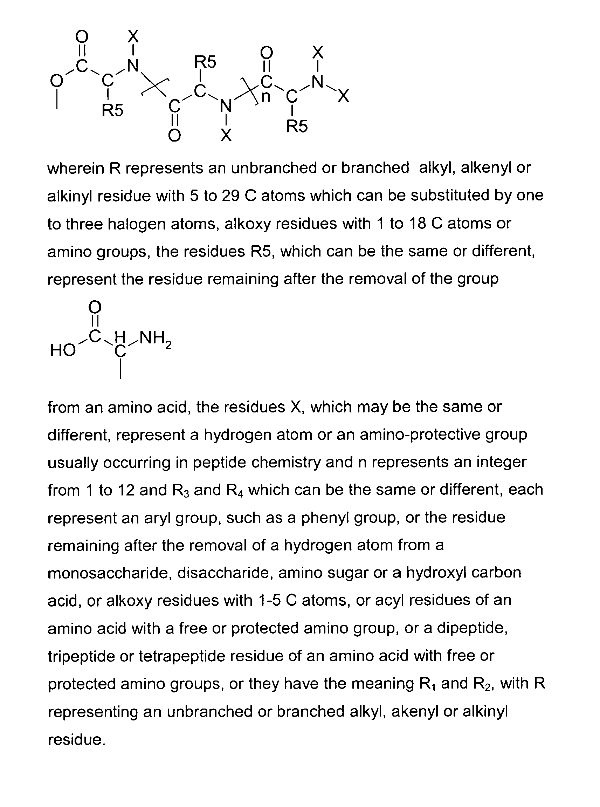

Unexpectedly, these compounds and derivatives of the general formula (I) form surprisingly stable carrier molecules and differ significantly from the siosomes and liposomes described in the prior art. Such vesicles are structurally distinct from the carrier systems of the prior art. Furthermore, it is a preferred embodiment of the invention to provide specific carrier systems for the intracellular "transfection" delivery of active substances as chemostatics, anti-cancer agents, such as doxorubicin, cis-platin, siRNA and nucleic acids, such as RNA, DNA, antiviral and antibacterial agents and active substances for CNS disorders, whereby one or more of the cationic lipids are covalently attached to the silicon and/or as a derivative of the chemical groups and/or residues of R₁, R₂, R₃ and R₄ as described in figure 2. These compounds according to the present invention are used as cationic sugar silanes and/or cationic sugar siosomes for the encapsulation, entrapment and/or complex formation of active substances. Unexpectedly, these compounds form stable carriers with high encapsulation efficiency and intracellular delivery of the active substances and specifically the nucleic acids, such as siRNA and anticancer, antiviral, antibacterial agents and therapeutics for the treatment of CNS disorders.

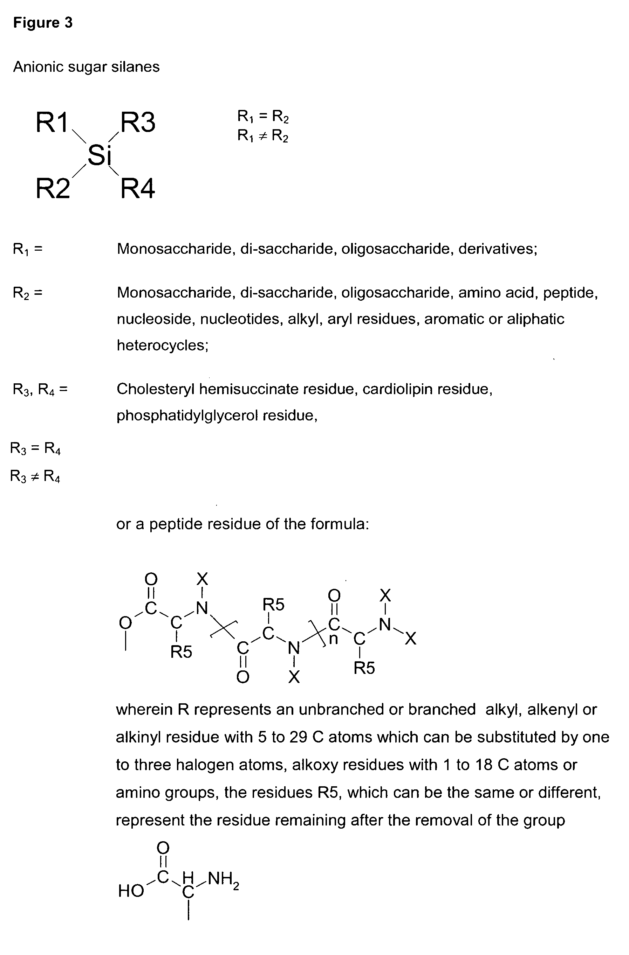

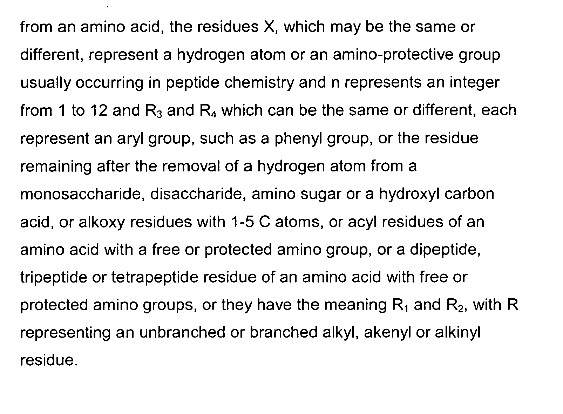

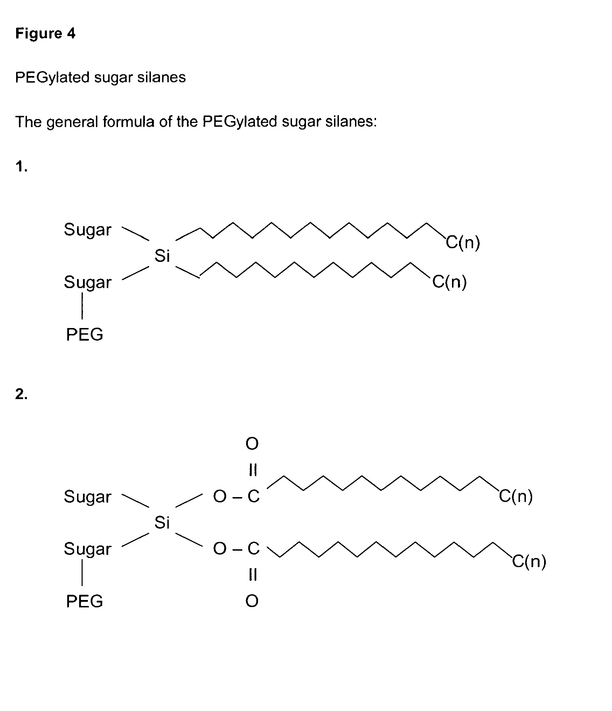

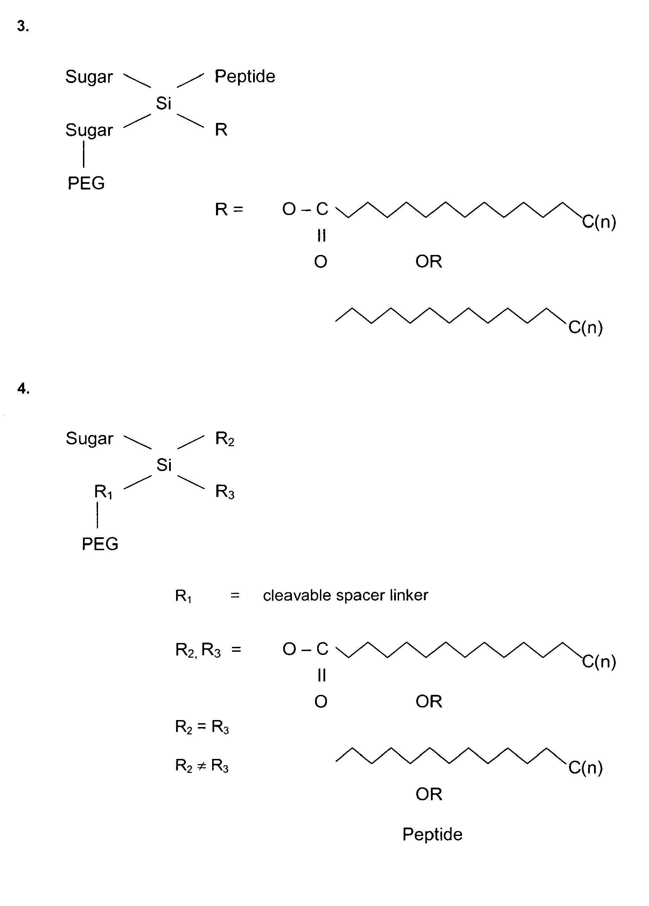

Furthermore, it is a preferred embodiment of the present invention to provide a carrier and intracellular delivery system for active substances whereby one or more of the anionic lipids such as cholesteryl hemisuccinate residues, cardiolipin and/or derivatives and/or other anionic groups are covalently linked to one or more of the groups R_{1,} R₂, R₃ and R₄ (figure 3) and/or to the silicone central atom of sugar silanes and/or other silanes/amino-sugar silanes according to the present invention.

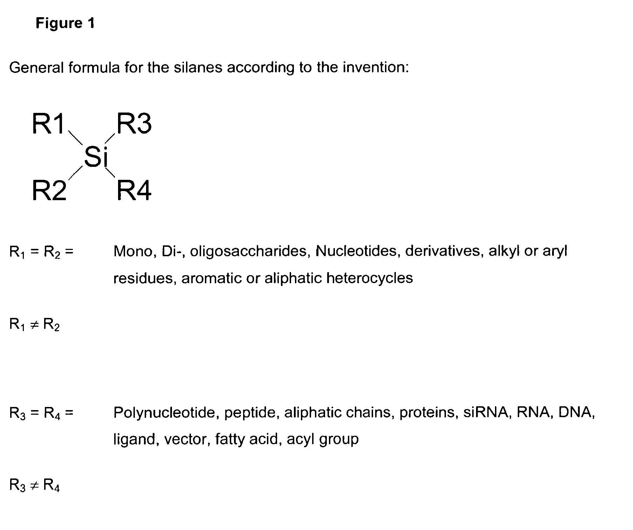

In yet another embodiment, the present invention provides a carrier and delivery system for active substances, whereby polyethylene-glycols of varying molecular weights can be used to be covalently attached to the silicone with or without spacer and/or chemical group or covalently attached to one or more of the groups R_{1,} R₂, R₃ and R₄ of the general formula according to figure 1. According to the present application, the covalent attachment can be carried out by means of a cleavable spacer linker.

As an example:

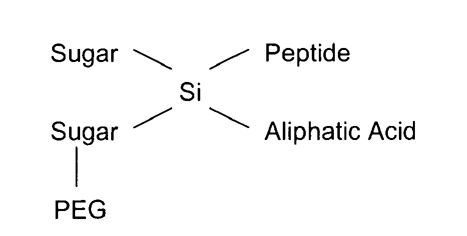

Furthermore, it is a preferred embodiment of the present invention that the method of preparation of the carrier system can be carried out by mixing the active substances with the polyethylene-glycols of varying molecular weights at selected pH and temperature with an appropriate solvent. The active substance-PEG mixture can be used for the preparation of the carrier and delivery system according to the procedures described in this invention.

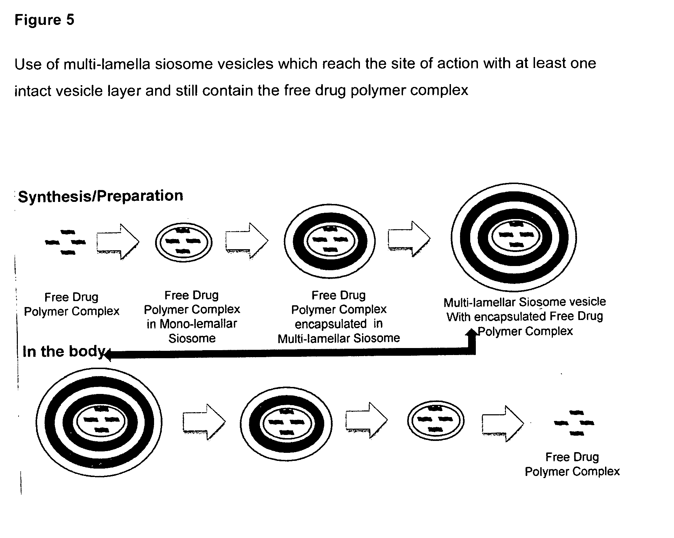

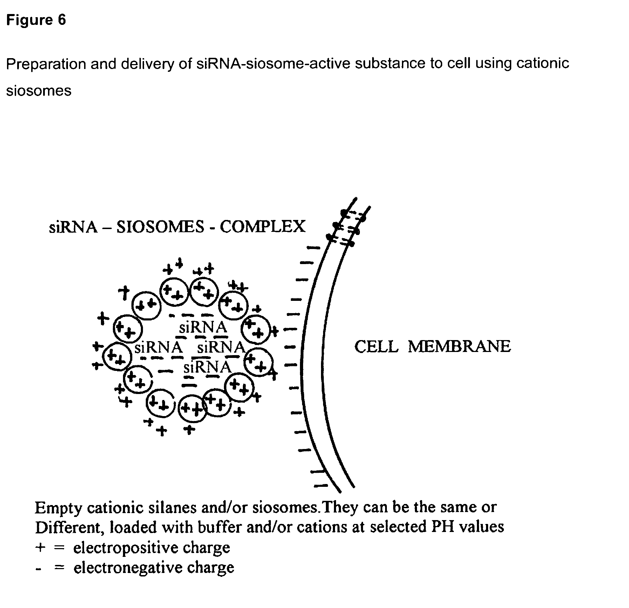

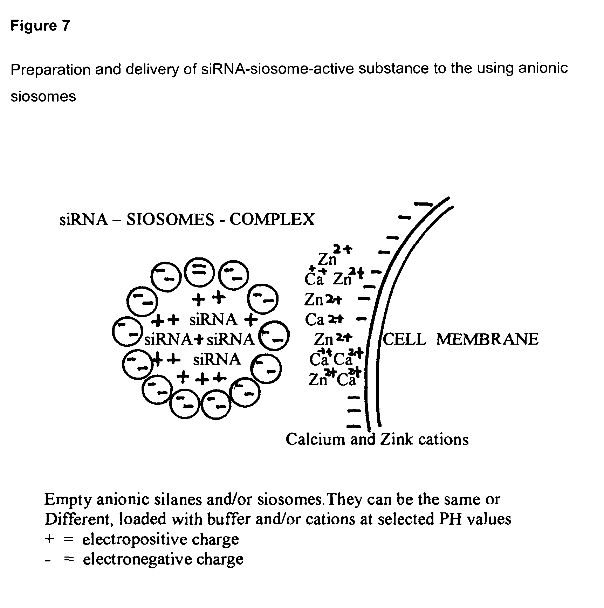

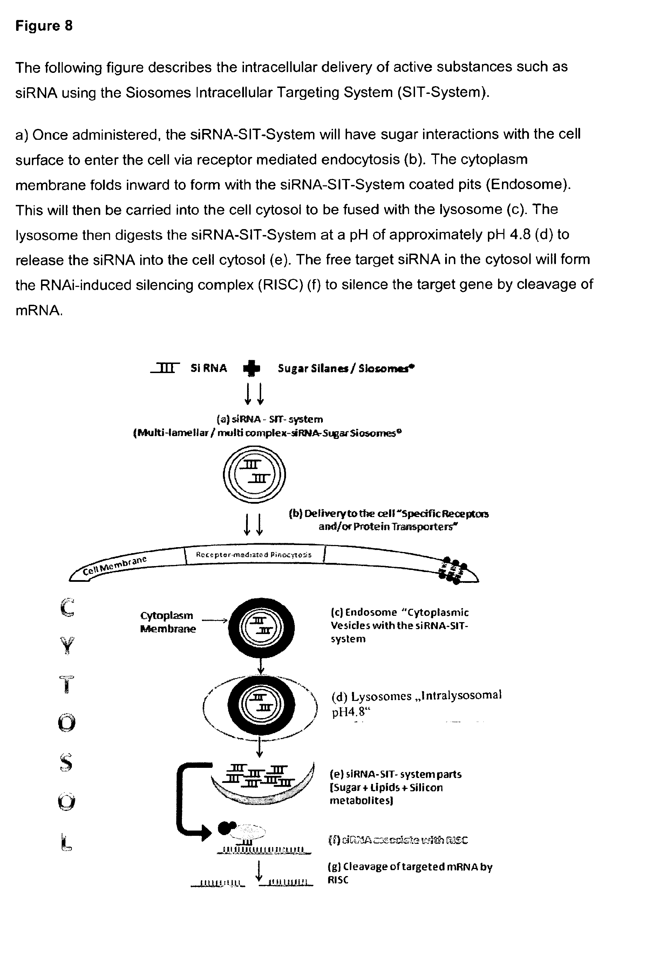

Surprisingly, preparation of a carrier system using these procedures produces stable vesicles with high encapsulation and entrapment efficiency and delayed release of the active substances in the target cells (Figures 5 ― 8).

In the method according to the invention, siosomes as carrier systems made from non-toxic lipids, peptides, carbohydrates, polymers, nucleosides and/or other ingredients are used, wherefore they are applicable for in vivo use in different therapeutics.

Furthermore, it is a preferred embodiment of the present invention that the active substance, such as nucleic acids, DNA, RNA, siRNA can be loaded with cations prior to the preparations of the siosomes carrier systems according to the procedures described in the examples of this invention (Figures 5 ― 8).

Furthermore, it is a preferred embodiment of the present invention that the cationic silanes and/or siosomes can be loaded with anionic salts for the preparation of the siosome carrier and targeting systems according to the procedures described in the examples of the present invention (Figures 5 ― 8).

Furthermore, it is a preferred embodiment of the present invention, that the anionic silanes and/or siosomes can be loaded with cationic salts for the preparation of the siosome carriers and intracellular targeting systems according to the procedures described in the examples of the present invention (Figures 5 ― 8).

Furthermore, it is a preferred embodiment of the present invention, that the siosomes carrier and targeting system can be comprised from one or more silanes according to the procedures described in the examples of the present invention.

It is a further preferred embodiment of the invention that the carrier and targeting systems can be prepared using silanes described in the present invention and lipids used for the preparation of the liposomes, such as anionic lipids, cationic lipids, and any other lipids known to person skilled in the art.

Unexpectedly, the above mentioned methods of preparations of the active substances, cationic, anionic silanes and/or siosomes and the mixed siosomes and lipo-sio-carrier systems are structurally distinct from the carrier systems of the prior art and exhibit adjustable encapsulation efficiency, release profile and transfection properties and efficiencies for the active substances.

It is a preferred embodiment of the present invention, that buffer solution containing TRIS, HEPES; MOPS, MES or other commonly used biological buffers will be used for the preparation of the siosome carrier and targeting system. The use of such buffer surprisingly enhances the efficiency, strength and stability of the active substance-siosome complex.

A preferred embodiment of the present invention is that the method for preparation of the carrier system includes one or more of the following additives, including detergent solution, adjuvant solution, salt solution, PEG-conjugates, cryoprotectants and/or other vesicles and ingredients.

In the method according to the invention, siosomes and/or siosomes-liposomes-complex (sio-lipo) may be administered as oral, rectal, vaginal, topical, nasal, intradermal, or parenteral, the term parenteral including subcutaneous, intravenous, intramuscular and infusion. This particular mode of administration will depend, of course, upon a particular drug selected, the severity of the condition being treated and the dosage required for the therapeutic efficacy.

The preferred organosilicon, sugar organosilicon, and amino sugar organosilicon compounds are listed in table 1.

**Table 1. Preferred organosilicon, sugar organosilicon, and amino sugar organosilicon compounds for carrier system production.**

| No. | Structure and Name | Molecular Formula | MW (Da) |
|---|---|---|---|
| Sil 1 | 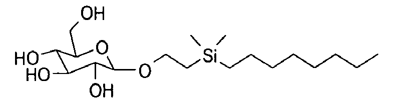 | | |
| | 2-(Dimethyloctylsilyl)ethyl-b-D-glucopyranosid | C18H3806Si | 378.59 |
| Sil 2 | 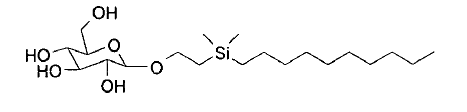 | | |
| | 2-(Dimethyldecylsilyl)ethyl-b-D-glucopyranosid | C20H4206Si | 406.64 |
| Sil 3 | 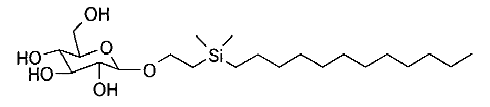 | | |
| | 2-(Dimethyldodecylsilyl)ethyl-b-D-glucopyranosid | C22H4606Si | 434.69 |
| Sil 4 | 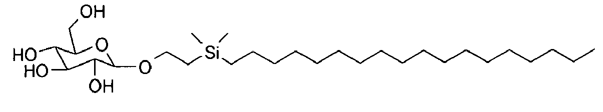 | | |
| | 2-(Dimethyloctadecylsilyl)ethyl-b-D-glucopyranosid | C28H58O6Si | 518.86 |
| Sil 5 | 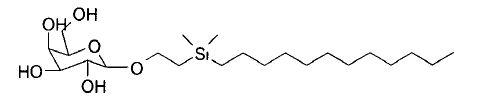 | | |
| | 2-(Dimethyldodecylsilyl)ethyl-b-D-galactopyranosid | C22H4606Si | 434,69 |
| Sil 6 | 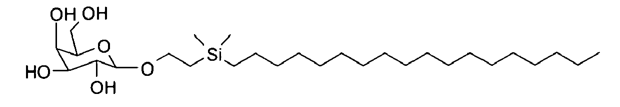 | | |
| | 2-(Dimethyloctadecylsilyl)ethyl-b-D-galactopyranosid | C28H5806Si | 518,86 |
| Sil 7 | 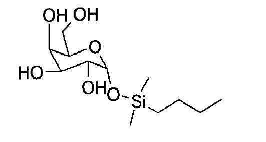 | | |
| | Butyldimethylsilyl-a-D-galactopyranosid | C12H2606Si | 294,42 |
| Sil 8 | 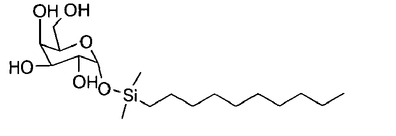 | | |
| | Decyldimethylsilyl-α-D-galactopyranosid | C18H3806Si | 378.59 |
| Sil 9 | 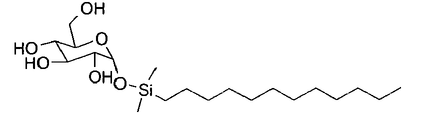 | | |
| | Dodecyldimethylsilyl-a-D-glucopyranosid | C20H4206Si | 406.64 |
| Sil 10 | 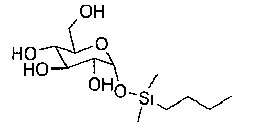 | | |
| | Butyldimethylsilyl-α-D-glucopyranosid | C12H2606Si | 294.42 |
| Sil 11 | 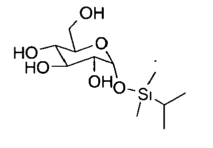 | | |
| | Dimethyl-isopropylsilyl-a-D-glucopyranosid | C11H2406Si | 280.40 |
| Sil 12 | 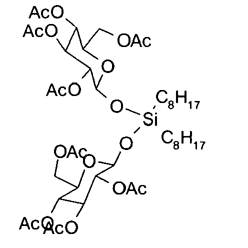 | | |
| | 1-O-Dioctylsilyl-di(2,3,4,6-O-tetraacetyl-b-D-glucopyranosid) | C44H72O20Si | 949.14 |
| Sil 13 | 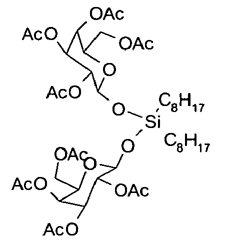 | | |
| | 1-O-Dioctylsilyl-di(2,3,4,6-O-tetraacetyl-b-D-galactopyranosid) | C44H72020Si | 949.14 |
| Sil 14 | 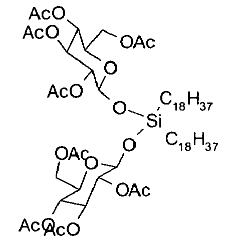 | | |
| | 1-O-Dioctadecylsilyl-di(2,3,4,6-O-tetraacetyl-b-D-glucopyranosid) | C64H112O20Si | 1229.68 |
| Sil 15 | 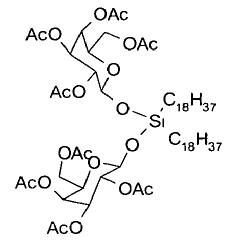 | | |
| | 1-O-Dioctadecylsilyl-di(2,3,4,6-O-tetraacetyl-b-D-galactopyranosid) | C64H112020S i | 1229.68 |
| Sil 16 | 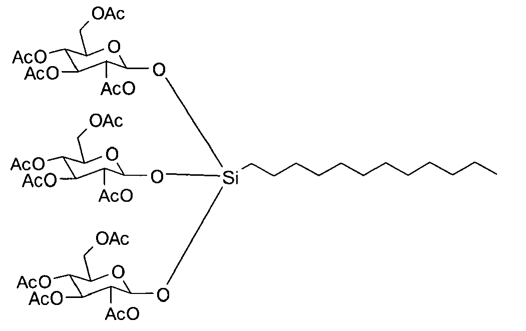 | | |
| | Dodecylsilyl-tris(2,3,4,6-O-tetraacetyl-b-D-glucopyranosid) | C54H82030Si | 1239.32 |
| Sil 17 | 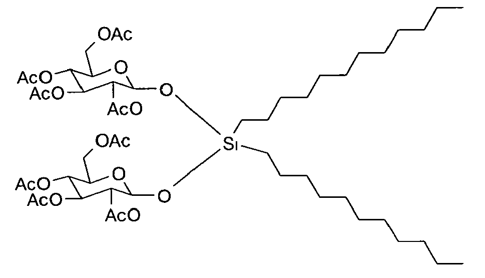 | | |
| | Didoceclylsily)-di(2,3,4,6-O-tetraacetyl-b-D-glucopyranosid) | C52H88020Si | 1061.36 |
| Sil 18 | 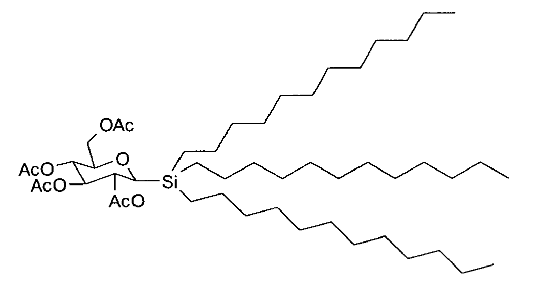 | | |
| | Tridodecylsilyl-(2,3,4,6-O-tetraacetyl-b-D-glucopyranosid) | C50H94O10Si | 883.39 |
| Sil 19 | 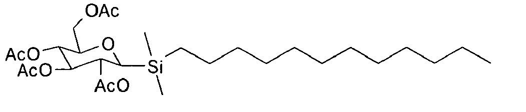 | | |
| | 1-O-Dimethyl(dodecyl)silyl-(2,3,4,6-O-tetraacetyl-b-D-glucopyranosid) | C28H50010Si | 574.79 |
| Sil 20 | 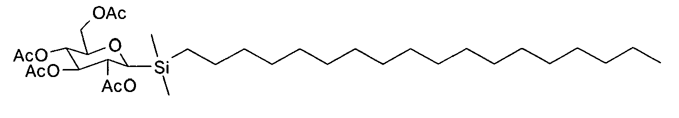 | | |
| | 1-O-Dimethyl(octadecyl)silyl-(2,3,4,6-O-tetraacetyl-b-D-glucopyranosid) | C34H62010Si | 658.95 |
| Sil 21 | 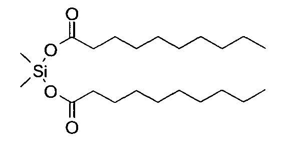 | | |
| | Di(decanoyloxy)dimethylsilan | C22H4404Si | 400.68 |
| Sil 22 | 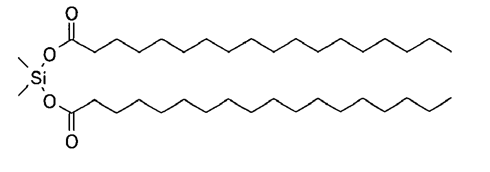 | | |
| | Di(octadecanoyloxy)dimethylsilan | C38H7604Si | 625.11 |
| Sil 23 | 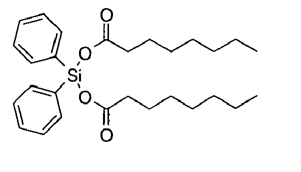 | | |
| | Di(octanoyloxy)diphenylsilan | C28H4004Si | 468.71 |
| Sil 24 | 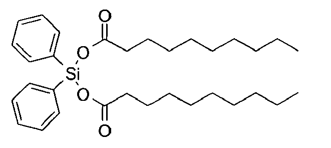 | | |
| | Di(decanoyloxy)diphenylsilan | C32H4804Si | 524.82 |
| Sil 25 | 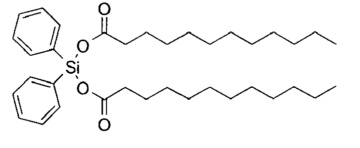 | | |
| | Di(dodecanoyloxy)diphenylsilan | C36H5604Si | 580.93 |
| Sil 26 | 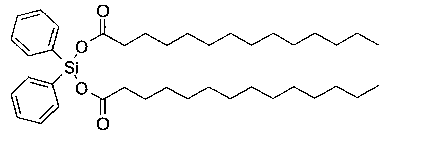 | | |
| | Di(tetradecanoyloxy)diphenylsilan | C40H6404Si | 637.04 |
| Sil 27 | 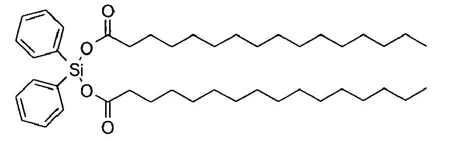 | | |
| | Di(hexadecanoyloxy)diphenylsilan | C44H7204Si | 693.15 |
| Sil 28 | 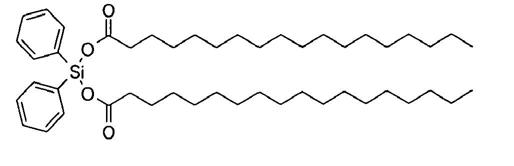 | | |
| | Di(octadecanoyloxy)diphenylsilan | C48H8004Si | 749.26 |
| Sil 29 | 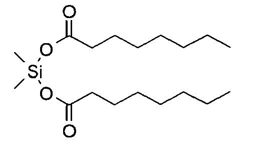 | | |
| | Di(octanoyloxy)dimethylsilan | C18H3604Si | 344.57 |
| Sil 30 | 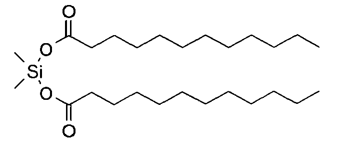 | | |
| | Di(dodecanoyloxy)dimethylsilan | C26H5204Si | 456.79 |
| Sil 31 | 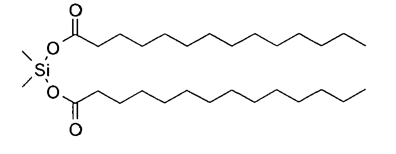 | | |
| | Di(tetradecanoyloxy)dimethylsilan | C30H6004Si | 512.90 |
| Sil 32 | 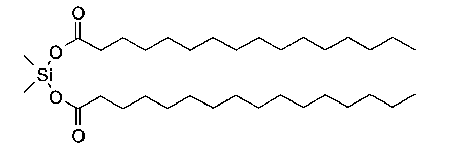 | | |
| | Di(hexadecanoyloxy)dimethylsilan | C34H6804Si | 569.00 |
| Sil 33 | 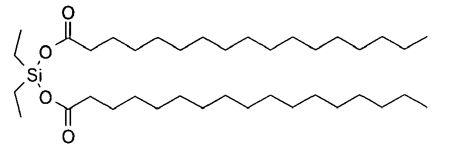 | | |
| | Di(heptadecanoyloxy)diethylylsilan | C38H7604Si | 625.11 |
| Sil 34 | 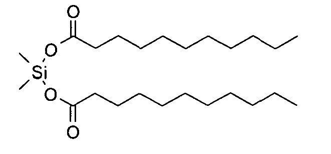 | | |
| | Di(undecanoyloxy)dimethylsilan | C24H4804Si | 428.73 |
| Sil 35 | 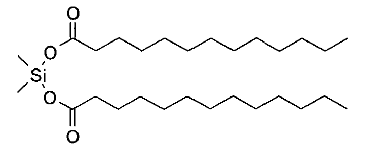 | | |
| | Di(tridecanoyloxy)dimethylsilan | C28H5604Si | 484.84 |
| Sil 36 | 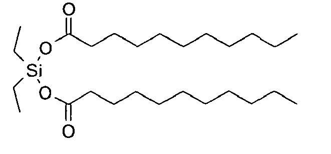 | | |
| | Di(undecanoyloxy)diethylylsilan | C26H5204Si | 456.79 |
| Sil 37 | 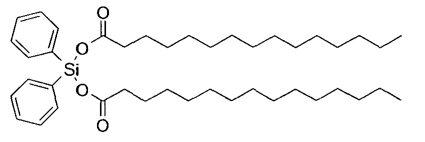 | | |
| | Di(pentadecanoyloxy)diphenylsilan | C42H6804Si | 665.09 |
| Sil 38 | 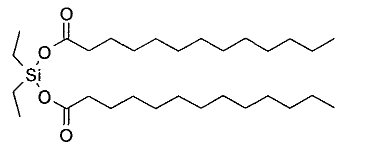 | | |
| | Di(tridecanoyloxy)diethylylsilan | C30H6004Si | 512.90 |
| Sil 39 | 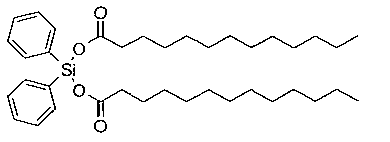 | | |
| | Di(tridecanoyloxy)diphenylsilan | C38H6004Si | 608.99 |
| Sil 40 | 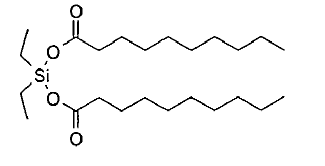 | | |
| | Di(decanoyloxy)diethylylsilan | C24H4804Si | 428.73 |
| Sil 41 | 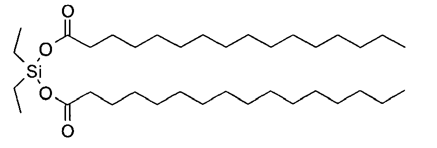 | | |
| | Di(hexadecanoyloxy)diethylylsilan | C36H7204Si | 597.06 |
| Sil 42 | 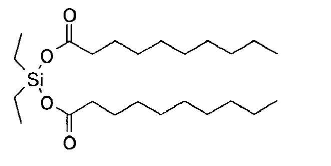 | | |
| | Di(decanoyloxy)diethylylsilan | C24H4804Si | 428.73 |
| Sil 43 | 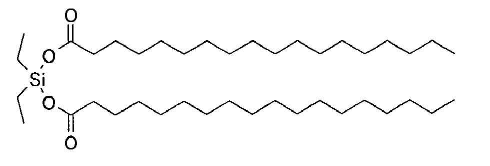 | | |
| | Di(octadecanoyloxy)diethylylsilan | C40H8004Si | 653.17 |
| Sil 44 | 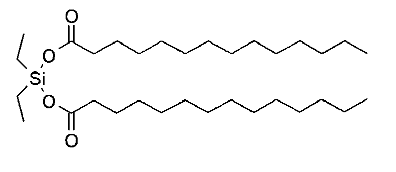 | | |
| | Di(tetradecanoyloxy)diethylylsilan | C32H6404Si | 540.95 |
| Sil 45 | 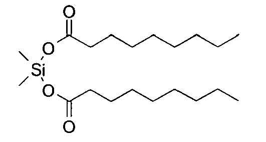 | | |
| | Di(nonanoyloxy)dimethylsilan | C20H4004Si | 372.63 |

### DEFINITIONS:

The following terms are defined as follows.

"Silane" is a chemical compound containing silicone, particularly with the chemical formula SiH₄, although other silicon-containing compounds may also be referred to herewith.

"Organosilicons" are organic compounds containing carbon-silicon bonds (C-Si).

"Sugar organosilicons" are organic compounds containing carbon-silicon bonds (C-Si) and at least one sugar group.

"Amino sugar organosilicons" are organic compounds containing carbon-silicon bonds (C-Si), at least one sugar group and one amino acid group. Examples of amino sugars and derivatives are well known in the art as glucosamine, galactosamine, mannosamine, neuramine acid, muramine acid, N-acetylglucosamine, further examples include acylated sugars and aminosugars. Amino sugar organosilicons comprise residues such as glucosamine, N-acetyl glucosamine, sialic acid or galactosamine, where the amino sugar residues can be the same or different, and represent the removal of a hydrogen from monoaminosaccharide and/or diaminosaccharide and/or polysaccharide.

"Siloxanes" are a class of organic or inorganic chemical compounds of silicon, oxygen, and usually carbon and hydrogen, based on the structural unit R₂SiO, where R is an alkyl group, usually methyl.

"Siosomes" are the vesicles created from organosilicon compounds. Siosomes consist of at least one concentric, self-contained layer of organosilicon compounds with the organosilicon general structure. An aqueous compartment is enclosed by the bimolecular organosilicon membrane.

"Sugar-siosomes" refer to siosomes consisting of sugar organosilicon compounds.

As referred to in this patent "blank siosomes" refers to any and all vesicles prepared from organosilicon compounds without encapsulated and/or entrapped pharmacologically and/or immunologically active agents. Specific examples of "blank siosomes" are siosomes filled with water, salts or buffer.

"Liposomes" are the vesicles created from phospholipids. Liposomes consist of at least one concentric, self-contained layer of phospholipids and an aqueous compartment enclosed by the bimolecular phospholipid membrane.

"Encapsulations" are to be understood as the capture by a siosome or liposome of a dissolved hydrophilic solute within the region of aqueous solution inside a hydrophobic membrane, whereby the dissolved hydrophilic solutes cannot readily pass through the lipid bi-layer.

"Entrapments" are to be understood as the capture by a liposome or siosome of hydrophobic solutes dissolved in the hydrophobic region of the bi-layer membrane.

"Combinations" or "complex combinations" of the present invention are intended to be understood as complexes formed between the organosilicon, sugar organosilicon, amino sugar organosilicon compounds, their derivatives, salts and/or the vesicles formed from them, with the biological agents, selected from antigens, pre-antigens, antigen conjugates, antibodies, pre-antibodies, antibody conjugates, allergens, allergen extracts, nucleic acids, plasmids, proteins, peptides, pharmaceutical agents, immunologically active substances and/or cosmetics, whereby the complexes are held together via the sum force of non-covalent bonds including Hydrophobic interactions between non-polar regions, such as the hydrophobic fatty acid chains of the organosilicon, sugar organosilicon, amino sugar organosilicon compounds and siosomes, non-specific protein-protein interactions between protein segments of an allergen and/or the peptide residues of the organosilicon, sugar organosilicon, amino sugar organosilicon compounds, protein-saccharide interactions, Van der Waals interactions including, dipole-dipole interactions, dipole-induced dipole interactions, dispersion forces (London forces), electrostatic interactions between charged resides, for example peptide residues and organosilicon, sugar organosilicon, amino sugar organosilicon compounds, and salts, ion-dipole forces; for example those between the salts of the buffer used in the preparation of the complex and H₂O, and ion-ion forces; for example those involving the salts of the buffer solution.

An "antigen" is to be understood as a substance that prompts the generation of antibodies and can cause an immune response.

A "pre-antigen" is to be understood as an antigen in an inactive state prior to processing to the active form.

An "antigen conjugate" is to be understood as an antigen conjugated covalently to another biological agent, such as an enzyme or other protein, or organosilicon molecule.

A "pre-antibody' is to be understood as an antibody in an inactive state prior to processing to the active form.

An "antibody conjugate" is to be understood as an antibody conjugated covalently to another biological agent, such as an enzyme or other protein, or organosilicon molecule.

The term "allergen" refers to a substance, protein or non-protein, capable of inducing allergy or specific hypersensitivity or an extract of any substance known to cause allergy. Almost any substance in the environment can be an allergen. The list of known allergens includes plant pollens, spores of mold, food preservatives, dyes, drugs, inorganic chemicals and vaccines. Allergens can enter the body by being inhaled, swallowed, touched or injected. Following primary exposure to an allergen, subsequent exposures result in hypersensitivity (allergic) reactions which may be immediate or delayed, local or systemic and include anaphylaxis and contact dermatitis.

The term "allergen extract" refers to a natural extract of multiple allergens, protein or non-protein, capable of inducing allergy or specific hypersensitivity or an extract of any substance known to cause allergy.

A "pharmaceutical agent" is to be understood as any medicament, intended for use in the diagnosis, cure, mitigation, treatment, or prevention of disease in humans or animals.

An "immunologically active substance" is to be understood as any substance that leads to an immune response in a human or animal patient.

As used herein "sugar" includes any and all monosaccharide, disaccharide, polysaccharide, amino-sugar or hydroxyl carbon acid, it's derivatives, salts and/or residues remaining after the removal of a hydrogen atom from it. The following are suitable examples of suitable monosaccharides; pentoses such as arabinose, ribose and xylose as well as hexoses such as glucose, mannose, galactose and fructose. Suitable amino sugars include e.g. glucosamin and galactosamin. A suitable carbon acid for example is glucronic acid. The hydroxyl carbon acids' hydroxyl groups can be free, partially derivatized or fully derivatized (protective groups) specific examples of amino sugar silicon compounds are listed herein.

A "patient" for the purposes of the present invention includes both humans and other animals, particularly mammals, and other organisms. Thus the methods are applicable to both human therapy, vaccinations, and veterinary applications. In the preferred embodiment the patient is a mammal, the most preferred being a human.

The term "animal" refers to an organism with a closed circulatory system of blood vessels and includes birds, mammals and crocodiles. The term "animal" used here also includes human subjects.

An "immunologically effective amount" is the quantity of a compound, composition or carrier system of the present invention which is effective in yielding the desired immunologic response.

The terms "treating cancer," "therapy," and the like refer generally to any improvement in the mammal having the cancer wherein the improvement can be ascribed to treatment with the compounds of the present invention. The improvement can be either subjective or objective. For example, if the mammal is human, the patient may note improved vigour or vitality or decreased pain as subjective symptoms of improvement or response to therapy. Alternatively, the clinician may notice a decrease in tumour size or tumour burden based on physical exam, laboratory parameters, tumour markers or radiographic findings. Some laboratory obtained results which the clinician may observe to check for any response to therapy include normalization of tests such as white blood cell count, red blood cell count, platelet count, erythrocyte sedimentation rate, and various enzyme levels. Additionally, the clinician may observe a decrease in a detectable tumour marker(s). Alternatively, other tests can be used to evaluate objective improvement such as sonograms, nuclear magnetic resonance testing and positron emissions testing.

"Inhibiting the growth of tumour cells" can be evaluated by any accepted method of measuring whether growth of the tumour cells has been slowed or diminished. This includes direct observation and indirect evaluation such as subjective symptoms or objective signs as discussed above.

Accordingly, the carrier systems of the invention are administered to cells, tissues, healthy volunteers and subjects and/or patients. Herein what is meant by "administered" is the administration of a therapeutically effective dose of the candidate agents of the invention to a cell either in cell culture or in a patient. Herein what is meant by "therapeutically effective dose" is a dose that produces the effects for which it is administered. The exact dose will depend on the purpose of the treatment, and will be ascertainable by one skilled in the art using known techniques. As is known in the art, adjustments for systemic versus localized delivery, age, body weight, general health, sex, diet, time of administration, drug interaction and the severity of the condition may be necessary, and will be ascertainable with routine experimentation by those skilled in the art. Herein what is meant by "cells" is almost any cell in which mitosis or miosis can be altered.

Additional "carriers" may be used in the "carrier system" of the present invention, and include any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the carrier systems.

The phrase "pharmaceutically-acceptable" refers to molecular entities and compositions that do not produce an allergic or similar untoward reaction when administered to a human. The preparation of an aqueous composition that contains a protein as an active ingredient is well understood in the art. Typically, such compositions are prepared as injectables, either as liquid solutions or suspensions; solid forms suitable for solution in, or suspension in, liquid prior to injection can also be prepared. The preparation can also be emulsified.

The term "pharmaceutically acceptable salt" refers to those salts of compounds which retain the biological effectiveness and properties of the free bases and which are obtained by reaction with inorganic acids such as hydrochloric acid, hydrobromic acid, sulphuric acid, nitric acid, phosphoric acid, methanesulphonic acid, ethanesulphonic acid, toluenesulphonic acid, salicylic acid and the like. Pharmaceutically acceptable salts include alkali metal salts, such as sodium and potassium, alkaline earth salts and ammonium salts.

Therefore, as used herein, "cancer" refers to all types of cancer or neoplasm or malignant tumours found in mammals, including carcinomas and sarcomas. Examples of cancers are cancer of the brain, breast, cervix, colon, head & neck, kidney, lung, non-small cell lung, melanoma, mesothelioma, ovary, sarcoma, stomach, uterus and Medulloblastoma.

The present invention relates to the use of organosilicon, sugar organosilicon, amino sugar organosilicon compounds, their derivatives, salts and/or the vesicles formed from them in a carrier system with antigens, pre-antigens, antigen conjugates, antibodies, antibody conjugates, allergens, allergen extracts, nucleic acids, plasmids, proteins, peptides, pharmaceutical agents, immunologically active substances and/or cosmetics, for the manufacturing of a pharmaceutical, immunological and/or cosmetic composition for the different indications.

The pharmaceutical composition of the invention optionally comprises of one or more pharmaceutically acceptable adjuvants, excipients, carriers, buffers, diluents and/or customary pharmaceutical auxiliary substances. The composition of the invention is administered in a pharmaceutically acceptable formulation. The present invention pertains to any pharmaceutically acceptable formulations, such as synthetic or natural polymers in the form of macromolecular complexes, nanocapsules, microspheres, or beads, and lipid-based formulations including oil-in-water emulsions, micelles, mixed micelles, synthetic membrane vesicles, and resealed erythrocytes. In addition to the said composition and the pharmaceutically acceptable polymer, the pharmaceutically acceptable formulation of the invention can comprise additional pharmaceutically acceptable carriers and/or excipients. As used herein, a pharmaceutically acceptable carrier includes any and all solvents, dispersion media, coatings, antibacterial and anti fungal agents, isotonic and absorption delaying agents, and the like that are physiologically compatible. For example, the carrier can be suitable for injection into the cell, tissues, organs and/or blood. Excipients include pharmaceutically acceptable stabilizers and disintegrants. In another embodiment, the pharmaceutically acceptable formulations comprise lipid-based formulations. Any of the known lipid-based drug delivery systems can be used in the practice of the invention. For instance, multivesicular liposomes (MVL), multilamellar liposomes (also known as multilamellar vesicles or MLV), unilamellar liposomes, including small unilamellar liposomes (also known as unilamellar vesicles or SUV), large unilamellar liposomes (also known as large unilamellar vesicles or LUV), multivesicular siosomes (MVS), multilamellar siosomes (MLS), unilamellar siosomes including small unilamellar siosomes can all be used so long as a sustained release rate of the carrier system composition of the invention can be established.

### pH-sensitive silane lipid:

The siosomes include a pH-sensitive lipid, that is a silane-lipid that forms bilayer vesicles in the absence of stabilising components only at specific pH ranges. These lipids are typically amphipathic lipids having hydrophobic and polar head group molecules, and when arranged into a bilayer are oriented such that the hydrophobic moiety is in contact with the interior, hydrophobic region of the bilayer membrane, and the polar head group moiety is oriented toward the exterior, polar surface of the membrane.

The pH-sensitive amphipathic lipids preferably have two hydrocarbon chains, typically acyl chains between about 8-22 carbon atoms in length, and have none or varying degrees of unsaturation.

### Polymer-derivatised silane-lipid component:

The siosomes also include a lipid derivatised with a hydrophilic polymer. The polymer derivatised lipids in the siosomes serve to stabilise the pH-sensitive silane-lipid to facilitate bilayer and siosome formation and to form a coating of polymer chains over the siosome surface to extend the blood circulation lifetime of the siosomes.

The hydrophilic polymer coating provides colloidal stability and serves to protect the siosomes from uptake by the mononuclear phagocyte system, providing a longer blood circulation lifetime for the siosomes to distribute in the organism. The polymer chains are attached to the lipid of the silane by a releasable bond for cleavage and release of the polymer chains in order to restore the pH-sensitivity of the siosomes.

Preferably, the derivatisable silane-lipid is a non-pH-sensitive siosome-forming amphipathic silane which can spontaneously form into a bilayer vesicle in water.

Hydrophilic polymers suitable for dervatising the amphipathic lipids include polyethyleneglycol, polyvinylpyrrolidone, polyvinylmethylether, polymethyloxazoline, polyethyloxazoline, polyhydroxypropyloxazoline, polyhydroxypropylene, methacrylamide, polymethacrylamide, polydimethylacrylamide, polyhydroxypropylmethacrylate, polyhydroxyethylacrylate, hydroxymethylcellulose, hydroxyethylcellulose, and polyaspartamide.

In a preferred embodiment, the hydrophilic polymer is polyethyleneglycol (PEG), preferably as a PEG chain having a molecular weight between 200 and 15,000 Dalton, more preferably between 500 and 5,000 Dalton.

### Siosome-entrapped active substance:

Entrapped in the siosomes is an active substance for intracellular delivery to the target cells. A variety of active substances can be entrapped in siosome vesicles, including water soluble agents, for example small water soluble organic compounds, peptides, proteins, DNA plasmids, oligonucleotides, and gene fragments, which can be stably encapsulated in the aqueous compartment of the siosomes. Furthermore, lipophilic compounds that stably partition in the lipid phase of the siosomes, or agents that can be stably attached, for example by electrostatic attachment to the outer siosome surfaces, are also intended.

The siosome-entrapped compound can also be an imaging agent for tracking progression of a disease. Imaging agents include chelates of radionuclides, such as technetium-99, indium-III, and iodine-125. The entrapped agent may also be a reporter molecule, such as an enzyme or a fluorophore, for use in in-vitro diagnostic assays.

The term "lipid" refers to any suitable material resulting in a bilayer such that a hydrophobic portion of the lipid material orients toward the bilayer, while a hydrophilic portion orients towards the aqueous phase.

The term "neutral lipid" refers to any of a number of lipid species which exist either in an uncharged or neutral zwitterionic form at physiological pH. Such lipids include, for example, diacylphophatidylcholine, diacylphosphatidylethanolamine, ceramide, sphingomyelin, cephalin and cerebrosides.

The term "non-cationic lipid" refers to any neutral lipid as described above, as well as anionic lipids. Examples of anionic lipids include cardiolipin, diacylphosphatidylserine and diacylphosphatidic acid.

The term "cationic lipid" refers to any of a number of lipid species which carry a net positive charge at physiological pH. Such lipids include, but are not limited to, DODAC, DOTMA, DDAB, DOTAP, DC-Chol and DMRIE.

Additionally, a number of commercial preparations of cationic lipids are available, which can be used in the present invention. These include, for example, LIPOFECTIN®, LIPOFECTAMINE® and TRANSFECTAM®.

### Active substances:

Many highly active and useful pharmaceutical agents suffer from suboptimal pharmacokinetics and/or biodistribution. Consequently, the therapeutic use of these pharmaceutical agents can be limited. Siosomes formulations of the invention can be used to improve the efficacy and toxicity profiles, and to improve the dosing schedule of the drug by modification of the pharmacokinetic and biodistribution properties. As used herein, the term active substance includes diagnostic agents.

The term active substance includes, but is not limited to, an analgesic, an anaesthetic, an anti-acne, an antibiotic, an antibacterial, an anticancer, an anticholinergic, an anticoagulant, an antidyskinetic, an antiemetic, an antifibrotic, an antifungal, an antiglaucoma agent, an anti-inflammatory, an antineoplastic, an antiosteoporotic, an anti-Parkinson's agent, an anti-sporatic, an antipyretic, an antithrombic, an anti-viral, a calcium regulator, a keratolytic, or a sclerosing agent.

### Anti-cancer active substances:

Doxorubicin hydrochloride is a drug used in cancer chemotherapy. It is an anthracycline antibiotic, closely related to the natural product daunomycin, and like all anthracyclines it works by DNA intercalation.

It is commonly used in the treatment of a wide range of cancers, including haematological malignancies, many types of carcinoma, and soft tissue sarcomas.

Doxorubicin hydrochloride has been used alone or in combination chemotherapy for the treatment of AIDS-related Kaposi's sarcoma and combination chemotherapy that includes the drug (doxorubicin, bleomycin, and vincristine) has been a preferred regimen, although many clinicians currently consider a liposomal anthracycline (doxorubicin or daunorubicin) the first-line therapy of choice for advanced AIDS-related Kaposi's sarcoma.

### EXAMPLES

The siosome containing preparations to be used in the method of the invention are applied in therapeutically acceptable amounts to produce therapeutically effective levels in the target. Such preparations for use may routinely contain known compatible carriers and other additives, such as buffering agents and preservatives, and optionally other active substances. Aqueous siosomal preparations may be formulated according to known methods, using suitable additives, such as dispersing or wetting agents and suspending agents. A sterile injectable preparation may be a sterile injectable solution or suspension in a non-toxic acceptable diluent or solvent, such as water, isotonic sodium chloride solution and buffer. In addition, sterile oils are often employed as a solvent or suspending medium.

The particular mode of administration selected will depend upon the particular selected active substance, the severity of the condition being treated and the dosage required for therapeutic efficacy. Such modes of administration include oral, rectal, intravaginal, topical, nasal, interdermal or parenteral routes, the term perenteral including subcutaneous, intravenous, intramuscular, intratumoural and infusion.

The carrier system according to the invention would preferably selectively release anti-cancer chemotherapeutics and any other active substances at intracellular targets at low pH-regions.

The examples are in no way intended to limit the scope of the invention.

Unless otherwise defined, all technical and scientific terms used herein have the same meaning as commonly understood by a person of ordinary skill in the art to which this invention belongs. Although methods and materials similar or equivalent to those described herein can be used in the practice or testing of the present invention, suitable methods and materials are described below.

Determining efficiency of carrier system protein complex formation with the organosilicon, sugar organosilicon, amino sugar organosilicon compounds and/or blank (unloaded) vesicles thereof

Carrier system formation efficiency, or protein complex formation between the organosilicon, sugar organosilicon, and/or amino sugar organosilicon compounds with biological protein agents, was calculated after centrifugation by quantification of the amount of the protein in the protein-complex fraction and the free non-complexed proteins presents in the aqueous supernatant phase. The protein was quantified using a modified Lowry method, Peterson G.L. 1983 "Determination of total protein", Methods Enzymology 91: 95-119.

### In-vivo protocol for the immunogenicity tests

Adult, pathogen-free New Zealand white rabbits were used in the immunogenicity tests. The rabbits were grouped (n=4) and immunized with the antigen and/or antigen mixture. Two sets of injections per rabbit on day 0 and 14. Intramuscular (i.m) or subcutaneous injections (s.c) were given in the hind legs following a standard protocol, with the first injection set given in the right leg, the second injection set given in the left leg. The injection sites were labelled for later identification. To assess immunogenicity, sera were prepared from blood samples obtained from each rabbit on day 0 (control) and day 19 (5days after the second injection). Blood (15ml per bleed) was collected from marginal ears veins using an 18 gauge needle, and then stored at 2-8 °C overnight to allow for clot shrinkage. The samples were then centrifuged (400x g) and the sera were removed by pipette and frozen as individual samples at -10°C to -25°C until assayed. The suspensions were injected with 1.0ml dose volumes.

### Homogenisation of the carrier systems

High pressure homogenisation using micro fluidiser was used for the preparation of the complex combinations, pressure range: 170-2700 bar.

### Lyophilisation/Dehydration of the carrier systems

The appropriate volume of suspension was added into each vial in 2 ml increments with vigorous vortexing between additions. Lyophilisation took place using freeze drying technology controlling all aspects of the lyophilisation cycle. The 1 shelf temperature ranges from -70°C to 60°C, process condenser temperature as low as -85°C and vacuum indication of 760 torr to 1 militorr. The dehydration of the carrier system without the use of a protective sugar depends on the organosilicon, sugar organosilicon and/or amino sugar organosilicon compound concentrations.

Dehydrated carrier system complexes are prepared by drying the preparations under reduced pressure in the presence of one or more protective sugars, e.g. disaccharides such as trehalose and sucrose. Sugar organosilicon or amino sugar organosilicon compounds could be used as protective compounds for dehydration and/or lyophilisation of the carrier systems.

The dehydration was performed to an end point which results in sufficient water being left in the preparation (e.g. at least 4-10 moles water/ silicon-lipid) so that the integrity of a substantial portion of the silicon-lipid combinations is retained upon rehydration.

### Hydration of the lyophilised carrier systems

Hydration is the final step in the preparation of the formulation for injection and/or other pharmaceutical formulations (for oral, nasal and/or topical administration). Typically, hydration solution is added in a series of aliquots to lyophilised carrier systems with vortex mixing after each addition of hydration media. Generally, water for injection (WFI) is used. Here, we also tested WFI that was supplemented with 5% ethanol (EtOH), which has the added advantage of enhancing hydration of the carrier systems.

### Extrusion of the carrier system suspensions

Virtually all organosilicon, sugar organosilicon and/or amino sugar organosilicon compound complexes with proteins, alleregens, and biological mixtures can be rapidly extruded resulting in a homogenous formulation of the preparations. Avestin LiposoFast and Northern Lipids Extruders (Thermobarrel Extruder) equipment attached to a nitrogen gas line was used.

### Preparation of dispersions

A dispersion is a homogenous mixture of substances that are not soluble in each other. Dispersion can be achieved by sonication, extrusion, high pressure homogenisation, shaking, freezing and thawing.

### Preparation of Organosilicons

Examples 1-3 describe the synthesis of a number of the organosilicons. This procedure was employed to prepare the organosilicons of interest.

### Preparation of amino sugar organosilicon compound

Example 4 describes the procedures used for the preparation of an example of the sugar organosilicons. Modified and/or different procedures have also been used for the synthesis of the other sugar organosilicon compounds.

### Preparation of siosomes and sugar siosomes

Example 5 describes the procedure used for the preparation of both blank (unloaded) siosomes and/or sugar-siosomes. The blank (unloaded) siosomes have water encapsulated inside the Siosomes and between the lipid layers.

All of the carrier systems and methods disclosed and claimed herein can be made and executed without undue experimentation in light of the present disclosure. While the carrier systems and methods of this invention have been described in terms of preferred embodiments, it will be apparent to those of skill in the art that variations may be applied to the carrier systems and methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

### Example 1: Preparation of Di(decanoyloxy)dimethylsilane

0.012 mol dimethyldichlorosilane is added to 50ml anhydrous ether, to which is further added 0.02 mol sodium decanoate under agitation at 40°C. To increase the yield, an excess of dimethyl dichlorosilane is added. This is followed by approximately another 3 hours of agitation at 40°C. For hydrolysis of the excess dimethyldichlorosilane, water is added and approximately 10 ml ether is applied for extraction 3 to 5 times. The combined ether extracts are dried over anhydrous sodium sulphate and, after filtering off, evaporated in a vacuum. The remaining di(decanoyloxy)dimethylsilane is recrystalised from heptane.

| | |
|---|---|
| Molecular formula: | C₂₂H₄₄O₄Si |
| Molecular mass: | 400.4gmol⁻¹ |
| Melting point: | 32-33°C |
| Yield | 92% |

### Example 2: Preparation of Di(octadecanoloxy)dimethylsilane

0.012 mol dimethyldichlorosilane is added to 50ml anhydrous ether, to which 0.02mol sodium octanoate is further added, under agitation at 40°C. This is followed by approximately 3 hours of further agitation at the same temperature. Disintegration of the excess dimethyldichlorosilane in water is carried out as per example 1. Recrystallisation from heptane is then performed.

| | |
|---|---|
| Molecular formula: | C₃₆H₇₆O₄Si |
| Molecular mass: | 624.7gmol⁻¹ |
| Melting point: | 62-64°C |
| Yield | 68% |

### Example 3: Preparation of Di(octanoloxy)diphenylsilane

0.01 mol dichlorophenyl saline is added to 50ml anhydrous ether, to which 0.02mol sodium octanoate is further added under agitation at 40°C. This is followed by approximately another 5 hours of agitation, after which water is added and approximately 10ml of ether is applied for extraction three times. The combined extracts are dried over sodium sulphate and evaporated in a vacuum.

| | |
|---|---|
| Molecular formula: | C₂₈H₄₀O₄Si |
| Molecular mass: | 468.4gmol⁻¹ |
| Melting point | 88-90°C |
| Yield | 88% |

### Example 4: Preparation of amino sugar organosilicon compound

Synthesis of tetradecyl-(2,3,4,5-tetra-O-acetyl-β-D-gluco-pyranosyloxy)silanes. There exist various possibilities for the formation of glucosidic bonds to the anomeric C-atom of the sugar, as described in the literature. To the most important variants belong to the Königs-Knorr reaction, the trichlorine-acetamidate-method and the TMSOTf method. All known methods are essentially S_{N}2-reactions, and through the right selection of the protective groups at the carbohydrate part and observance of suitable conditions of reaction, we obtained 1, 2-trans-tied-glucosides. A further requirement is that the anomeric C atom must be activated through a leaving group which determines the centre of the reaction. The aglycon must have one free hydroxyl group if necessary selectively non-blocked.

### Example 5: Preparation of siosomes

10µmol di(decanoyloxy)dimethylsilane is dissolved in 2ml ethanol, and small doses age gradually added over a period of 4 hours to an aqueous phase (volume 2ml) which can contain the compounds to be encapsulated and has been brought to a temperature of 80°C. Depending on the solubility of the compound to be encapsulated, it can also be a component of the organic phase. The material is the injected into pure water. In this case the agitation rate is 2000±200rpm. The encapsulated compounds are separated from the non-encapsulated compounds by dialysis, gel filtration or centrifuging, for example and the contents of the siosomes determined. The siosomes prepared in this manner had a uniform size of 300 to 500nm and are evidently all uni-lamellar. The siosomes stability was good.

The higher the number of side chain (from C=8 to C=18), the larger the siosomes become (200nm to 2µm). While in the case of di(octanoyloxy)dimethylsilane and di(decanoyloxy)dimethylsilane, nearly all the siosomes produced are unilamellar and of uniform size, other siosomes prepared according to the invention (with the number of carbon atoms in side chain C=12 to C= 18) have predominantly multilamellar structures. These siosomes have a size of up to 2µm.

### Example 6: Preparation of a carrier system complex of amino sugar organosilicon compound with insulin.

A dispersion of 10µmol of Didodecylsilyl-di(2,3,4,6-0-tetraacetyl-β-D-glucopyranosid) as the representative of amino sugar organosilicon compound, 0.01 M Tris/HCI, pH 7.4 and an aqueous solution of insulin (10µmol, volume 3ml) as antigen was prepared by mixing with a high pressure homogeniser. The homogenisation time may vary. The mixture was then incubated at 37°C for 30 minutes and sterile filtrated and lyophilised at the temperature of -70°C. The lyophilised insulin-amino sugar organosilicon complex was reconstituted by hydration in sterile (deionized) water and the carrier system solution was used for in-vitro investigations.

After reconstitution by rehydration, the said carrier system surprisingly retains in solution more than 95% of the biological activity of insulin. The yield of the preparation process resulted from 3 independent experiments, and was 80-90% of the starting concentrations. Stability tests performed at 0, 4, 8, 12 and 48 hours at 4°C, 25 °C and 37°C have shown that the carrier system complex of organosilicon-insulin is surprisingly stable and retains its biological activity.

### Example 7: Preparation of a carrier system comprising blank (unloaded) siosomes with antigens

A dispersion of lyophilised blank sugar-siosomes prepared from 2-(dimethyldodecosilyl)-2,3,4,6-tetra-o-acetyl-β-D-glucopyranosid and 0.01 M Tris/HCL, pH7.4 and an aqueous solution of the antigen mixture comprising of Cathepsin B (EC 3.4.22.1), MW 30kDa as Thiolproteinease and Cathepsin C (EC 3.4.14.1), MW 200kDa as multimeric, dipeptidyl peptidase 1 enzyme was prepared at 37°C by vortexing and high pressure homogenisation.

Directly afterward, the said dispersion was heated for 20 minutes at 25°C (until complete fluidisation of the blank siosome). The mixture was then lyophilised and stored at 4-8°C. The biological activities of Cathepsin B and C in the carrier system complex were determined after reconstitution of the lyophilised samples by hydration with sterile (deionized) water using Cathepsin Activity Assay Kit.

Surprisingly, the carrier system retains more than 95% of the biologically active principles in solution. The carrier system showed long-termed stability as a lyophilisate at 4-8°C. The use of simple procedures in creating carrier systems, with the antigens Cathepsin B and C as examples for proteins, with lyophilised blank (unloaded) sugar-siosomes, demonstrates the potential for scale-up preparation of larger amounts.

### Example 8:

A doxorubicin hydrochloride solution at 10 mg/ml was prepared using physiological saline. The predetermined amount of the doxorubicin hydrochloride solution was added to the siosome dispersion and the mixture was adjusted to pH 7.4, using 1 N NaOH or saturated sodium hydrogencarbonate and the mixture was stirred at 65°C for 30 minutes the thereby introduce the doxorubicin hydrochloride in the siosomes.

After doxorubicin hydrochloride introduction, the sample was cooled in an ice bath. Gel filtration was conducted using a column (Sepharose 4, fast flow, having a diameter of 2.8 cm and a length of 20 cm, fully substituted with 10% sucrose (pH 6.5)) to remove the doxorubicin hydrochloride that had not been introduced in the siosomes.

The amount of the sugar silanes in the siosomes, the amounts of the doxorubicin hydrochloride encapsulated in the siosomes and the siosomes themselves were measured by the same methods as in the above examples.

Concentration of the doxorubicin hydrochloride encapsulated in the siosomes was determined by measuring absorbance at 480 nm, using a spectrophotometer for the solution prepared by adding 2 ml of methanol to 40 µl of the doxorubicin siosomes. An alternative HPLC-based assay has also been used for the quantification of the doxorubicin in the formulations.

The siosome size was measured using Zetasizer (Malvern Instruments).

### Example 9:

Doxorubicin hydrochloride was dissolved in PEG 600 at a concentration of 10 mg/ml. Tris-buffer was added at pH 7.4 and the temperature was kept at 65°C for 30 minutes under stirring.

The sample was cooled in an ice bath. A dispersion of 10 µmol of Didodecylsilyldi(2,3,4,6-o-tetraacetyl-β-D-glucopyranosid) as the representatives of amino sugar organo-silicon compound and the doxorubicin hydrochloride-PEG 600 was prepared by mixing with a high speed homogeniser.

The homogenisation time may vary. The mixture was then incubated at 37°C for 30 minutes. The doxorubicin hydrochloride-PEG 600 that had not been introduced in siosomes has been removed, and the siosomes mixtures has been lyophilised at the temperature of -70°C.

The above procedures described in example 9 were employed to prepare carrier systems of doxorubicin hydrochloride using PEG 2000 in ethanol, PEG 4000 in ethanol and HEPES as buffer.

In addition, different ratios of doxorubicin hydrochloride : PEG : silanes have been used, for example at a ratio of 1 : 3 : 4.

### Example 10:

The above procedures described in example 8 were employed to prepare the carrier siosomes systems in this example. According to these methods of preparation, a dispersion of each of the following amino sugar organo-silicon compounds were prepared, using doxorubicin hydrochloride solutions at 10 µg/ml.
Sil8: Decyldimethylsilyl-α-D-galactopyranosid
Sil9: Dodecyldimethylsilyl-α-D-glucopyranosid
Sil14: 1-o-Dioctadecylsilyl-di(2,3,4,6-0-tetracetyl-β-D-glucopyranosid)
Sil15: 1-o-Dioctadecylsilyl-di(2,3,4,6-o-tetracetyl-β-D-galactopyranosid)
Sil17: Didodecylsilyl-di(2,3,4,6-o-tetracetyl-β-D-glucopyranosid)
Sil18: Tridodecylsilyl-(2,3,4,6-o-tetracetyl-β-D-glucopyranosid)
Sil22: Di(octadecanoyloxy)dimethylsilan

Each of the above mentioned silanes have been used for the encapsulation of the doxorubicin hydrochloride and the preparation of the lyophilisate according to the procedures described in this invention.

The ratio of doxorubicin Hcl to silanes were was follows:

| Doxorubicin Hcl | : | Sugar silane / silane |
|---|---|---|
| 1 | : | 1 |
| 1 | : | 3 |
| 1 | : | 5 |
| 1 | : | 10 |

The following parameters have been determined:

| | |
|---|---|
| 1 | Physico chemical properties |
| 2 | Encapsulated efficiency of doxorubicin |
| 3 | Stability |
| 4 | Particle size distribution prior to lyophilisation and after dehydration using sterile water for injection |
| 5 | Release profile |

### Example 11:

The above procedures described in example 10 have been employed to prepare the following combination siosomes. Formulations with doxorubicin hydrochloride:

| | |
|---|---|
| 1. | Sil8 + Sil17 |
| 2. | Sil9 + Sil17 |
| 3. | Sil14 + Sil17 |
| 4. | Sil8 + Sil18 |
| 5. | Sil8 + Sil17 + Sil22 |
| 6. | Sil8 + Sil15 + Sil17 |
| 7. | Sil18 |
| 8. | Sil22 |
| 9. | Sil8 + Sil22 |

The paramters described in example 10 have been determined using the standard procedures according to this invention. The ratio of doxorubicin hydrochloride to sugar silane / silane was as follows:

| Doxorubicin Hcl : | : | Sugar silane / silane "Total amount" |
|---|---|---|
| 1 | : | 1 |
| 1 | : | 3 |
| 1 | : | 5 |
| 1 | : | 10 |

### Example 12: Preparation of nucleic acids-siosomes complex

The preparation of the siosomes to be used according to the invention will be as mentioned in the examples. Briefly, the silanes and any further lipids to be used are mixed in a suitable solvent, for example in acetone, ethanol, and evaporated to dryness under a stream of nitrogen. To prepare the siosomes, the dried lipid mixture is hydrated in a buffer at selected pH, including short periodical vortexing, and sonicated at the end of the hydration period in order to form multilamellar siosomes.

Nucleic acid includes in this context DNA, RNA, siRNA, and oligonucleotides of DNA and RNA.

To prepare the nucleic acid-siosome complex, the nucleic acid and the lipids are each diluted in an appropriate solvent at selected pH, for example in serum-free DMEM (Dulbecco's Modified Eagle's Medium), mixed and preincubated for a suitable period of time. The amount of nucleic acid to lipid can varied, but a weight ratio between nucleic acid and lipid in the range of 0.10 : 10 to 3 : 4 has been found to be satisfactory.

Due to charge interactions, the nucleic acid forms a strong complex with the cationic siosomes surface after simple mixing of the components.

Good transfection results have been obtained in tests using 2 or 4 µg respectively of RNA to 20 to 40 µg of total lipid.

### Example 13: Preparation of nucleic acid-silane/siosome active substance

The procedures for the preparation of the nucleic acid-siosomes complex using cationic silanes, sugar silanes and/or cationic siosome, sugar siosomes according to the invention are briefly described.

Using cationic silanes and/or cationic siosomes:

The cationic silanes-lipid mixture and/or the empty cationic siosomes (the same or different siosomes prepared from the same or different silanes) loaded with buffer and/or cations (2⁺) such as Ca²⁺, Zn²⁺, Mg²⁺ at selected pH-values, is hydrated in an appropriate medium containing the nucleic acid.

The mixture will be vortexed and/or sonicated for a period of time at suitable temperature.

Procedures for characterisation, particle stirring, stability, release, lyophilisation, sterilisation, rehydration, storage, and administration were employed according to this invention.

The amount of nucleic acid to silanes/siosomes lipid can vary.

Using PEGylated cationic silanes and/or PEGylated cationic siosomes:

The above procedures as described in example 13 were employed to prepare carrier systems, comprising of PEGylated cationic organosilicon, sugar organosilicon, amino-sugar organosilicon compounds and/or PEGylated cationic siosomes with nucleic acids, such as DNA, RNA, siRNA and nucleotides.

Using cationic silanes and/or cationic siosomes and PEG:

The above procedures as described in example 13 were employed to prepare carrier systems, comprising of cationic organosilicon, sugar organosilicon, amino-sugar organosilicon compounds and/or cationic siosomes with nucleic acids, such as DNA, RNA, siRNA and PEG. The molecular weight of PEG can vary from 200 to 10,000 Daltons.

### Example 14: Preparation of nucleic acids-silane/siosomes active substances

The procedures for the preparation of the nucleic acid-siosome complex as active substance using anionic silanes, sugar silanes and/or anionic siosomes, sugar siosomes according to the invention are briefly described herein:

Nucleic acids (DNA, RNA, siRNA, nucleotides) are treated with silanes, sugar silanes and/or siosomes, sugar siosomes and/or with cationic silanes, sugar silanes and/or siosomes, sugar siosomes and/or cationic salts such as Zn2+, Ca2+, Mg2+, or Na+ and/or hydrophilic polymers such as PEG (MW 200 - 10000 daltons). The resulting nucleic acid-silane/siosome complex are then mixed with anionic silanes/sugar ailnaes, anionic siosomes/sugar siosomes and/or cationic salts such as Zn2+, Ca2+, Mg2+, or Na+. The resulting compound is a carrier system for administration of nucleic acid-silane/siosomes active substances.

Otherwise, the procedures described in the invention were employed for the preparation of the silanes, siosomes, complex characterisation, stability, release, particle sizing, sterilisation and rehydration according to the invention.

The ratio between the used nucleic acids, silanes, siosomes-lipids and/or other lipids, PEG, cations, can vary.

### Example 15: Preparation of active substance-liposomes-siosomes-complex (Lipo-Sio-Com lex

Lipid refers to any suitable material resulting in a bilayer. Lipids used for the preparation of the siosomes, such as phospholipids (phosphatiylethanolamines, phosphatidylcholine, phosphatidylinositol, phosphatidylserine), cationic lipids, such as DOTMA (Lipofectin™), DOSPA (LipofectAMINE™), DOTAP, DC-Chol, DOPE, GL-67™, and anionic lipids, such as cardiolipin (diphosphatidylglycerol) and/or cholesterylhemisuccinate will be used according to the invention in combination with the silanes, sugar silanes, amino-sugar silanes, anionic sugar silanes, cationic sugar silanes, and/or PEGylated silanes. Preparations of the siosomes-liposomes-active substance according to the invention are briefly described:

Lipids for the preparation of liposomes (A) are mixed with the silanes, sugar silanes, cationic, anionic silanes and/or PEGylated silanes (B), which results in a mixture of the lipids (A) and (B) in appropriate solvents, such as ethanol, acetone or chloroform. The mixture of the lipids occurs by vortex mixing under nitrogen, followed by the removal of residual solvent under high vacuum for a period of time. Lipids were hydrated with appropriate buffers containing the agent and/or active substances at selected pH, temperature and salt concentrations. Thereafter, separation of the unencapsulated agent, lyophilisation, dehydration, characterisation, particle sizing, release, stability, storage and administration can be performed according to the procedures of the invention and to any procedures common to those skilled in the art.

### Example 16: Preparation of siosomes with encapsulated liposomes

The procedures disclosed in this invention can also be applied for the encapsulation of liposomes within siosomes. Liposomes with encapsulated agents and/or active substances are prepared according to the known procedures for the liposomes or lipid bilayer preparations. Particle sizing, separation of the free (unencapsulated) agent, in-vitro stability, lyophilisation will be performed according to the standard methods and procedures of the invention.

Liposomes will be encapsulated in the siosomes prepared according to the procedures described in the invention. The liposomes-siosomes-active substance complex will be lyophilised according to the procedures of the present invention with a cryoprotectant.

Reconstitution is carried out to form a liposome-siosome-complex concentrate, using an appropriate buffer at a selected pH.

## Claims

1. Carrier and targeting system for the intracellular delivery and release of a siosome-and/or siosome-liposome-entrapped active substance, comprising organosilicon, sugar organosilicon, amino-sugar organosilicon, alkyl, alkoxy or acyloxy organosilicon, peptide organosilicon, amino acid(s) organosilicon, lipids and/or their derivatives.

2. Carrier and targeting system according to the preceding claim, **characterized in that** the organosilicon, sugar organosilicon, or amino-sugar organosilicon compounds are **characterized by** the general formula (I): 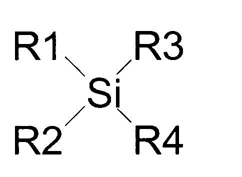 whereby R₁ and R₂ can be the same or different, and where R₃ and R₄ can be the same or different, whereby
R_{1,} R₂ = Mono, Di-, oligosaccharide, nucleotide, alkyl or aryl, aromatic or aliphatic heterocycles, and
R₃, R₄ = Polynucleotide, peptide, aliphatic chains, proteins, siRNA, RNA, DNA, ligand, vector, fatty acid, acyl group.

3. Carrier and targeting system according to any one of the preceding claims, **characterized in that** one or more cationic lipids are covalently attached to the silicon and/or as a derivative of the chemical groups and/or residues of R₁, R₂, R₃ and R₄ of the general formula (I), whereby
R₁ = Monosaccharide, di-saccharide, oligosaccharide, derivatives;
R₂ = Monosaccharide, di-saccharide, oligosaccharide, amino acid, peptide, nucleoside, nucleotides, alkyl, aryl residues, aromatic or aliphatic heterocycles;
R₃, R₄ = 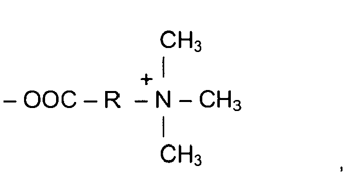 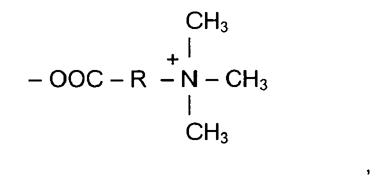 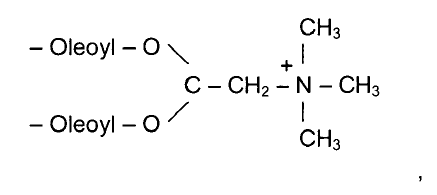
or a peptide residue of the formula: 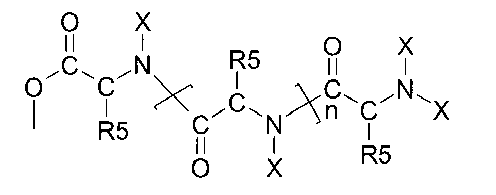 wherein R represents an unbranched or branched alkyl, alkenyl or alkinyl residue with 5 to 29 C atoms which can be substituted by one to three halogen atoms, alkoxy residues with 1 to 18 C atoms or amino groups, the residues R5, which can be the same or different, represent the residue remaining after the removal of the group 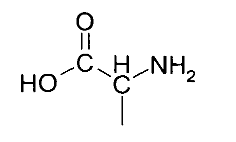 from an amino acid, the residues X, which may be the same or different, represent a hydrogen atom or an amino-protective group usually occurring in peptide chemistry and n represents an integer from 1 to 12 and R₃ and R₄ which can be the same or different, each represent an aryl group, such as a phenyl group, or the residue remaining after the removal of a hydrogen atom from a monosaccharide, disaccharide, amino sugar or a hydroxyl carbon acid, or alkoxy residues with 1-5 C atoms, or acyl residues of an amino acid with a free or protected amino group, or a dipeptide, tripeptide or tetrapeptide residue of an amino acid with free or protected amino groups, or they have the meaning R₁ and R₂, with R representing an unbranched or branched alkyl, akenyl or alkinyl residue.

4. Carrier and targeting system according to any one of the preceding claims, **characterized in that** one or more anionic lipids, such as cholesteryl hemisuccinate residues, cardiolipin and/or their derivatives and/or other anionic groups, are covalently linked to the silicone central atom of the silanes, sugar silanes and/or amino-sugar silanes and/or to one or more of the groups R₁, R₂, R₃ and R₄ of the general formula (I), whereby
R₁ = Monosaccharide, di-saccharide, oligosaccharide, derivatives;
R₂ = Monosaccharide, di-saccharide, oligosaccharide, amino acid, peptide, nucleoside, nucleotides, alkyl, aryl residues, aromatic or aliphatic heterocycles;
R₃, R₄ = Anionic Lipid, cholesteryl hemisuccinate residue, cardiolipin residue, phosphatidylglycerol residue,
or a peptide residue of the formula: 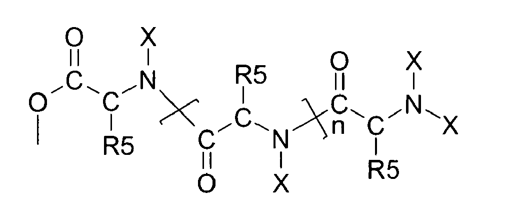 wherein R represents an unbranched or branched alkyl, alkenyl or alkinyl residue with 5 to 29 C atoms which can be substituted by one to three halogen atoms, alkoxy residues with 1 to 18 C atoms or amino groups, the residues R5, which can be the same or different, represent the residue remaining after the removal of the group 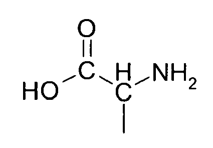 from an amino acid, the residues X, which may be the same or different, represent a hydrogen atom or an amino-protective group usually occurring in peptide chemistry and n represents an integer from 1 to 12 and R₃ and R₄ which can be the same or different, each represent an aryl group, such as a phenyl group, or the residue remaining after the removal of a hydrogen atom from a monosaccharide, disaccharide, amino sugar or a hydroxyl carbon acid, or alkoxy residues with 1-5 C atoms, or acyl residues of an amino acid with a free or protected amino group, or a dipeptide, tripeptide or tetrapeptide residue of an amino acid with free or protected amino groups, or they have the meaning R₁ and R₂, with R representing an unbranched or branched alkyl, akenyl or alkinyl residue.

5. Carrier and targeting system according to any one of the preceding claims, **characterized in that** the encapsulated and/or entrapped active substance is an anti-cancer agent, such as doxorubicin, cis-platin, carbo-platin and/or oxali-platin.

6. Carrier and targeting system according to any one of the preceding claims, **characterized in that** the encapsulated and/or entrapped active substance is genetic material, such as DNA, RNA, siRNA and/or an anti-viral and/or anti-bacterial agent.

7. Carrier and targeting system according to any one of the preceding claims, **characterized in that** polyethylene-glycol is covalently attached to the silicone and/or one or more of the groups R_{1,} R₂, R₃ and R₄ of the general formula (I).

8. Carrier and targeting system according to any one of the preceding claims, **characterized in that** the covalent attachment of polyethylene-glycol can be carried out by means of a cleavable spacer linker.

9. Carrier and targeting system according to any one of the preceding claims, **characterized in that** the composition is lyophilized.

10. Carrier and targeting system according to any one of the preceding claims, **characterized in that** the composition comprises a cryoprotectant.

11. Carrier and targeting system according to any one of the preceding claims, **characterized in that** the cryoprotectant is a disaccharide selected from the group consisting of sucrose, maltose, trehalose, and/or lactose.

12. Carrier and targeting system according to any one of the preceding claims, **characterized in that** the cryoprotectant is a disaccharide having a concentration of 1-40%.

13. Carrier and targeting system according to any one of the preceding claims, comprising additionally of liposomes, unsaturated lipids, a vesicle-forming lipid, a hydrophilic polymer and/or liposomes.

14. Carrier and targeting system according to any one of the preceding claims, **characterized in that** the vesicle-forming lipids exhibit two hydrocarbon chains, typically acyl chains, and a polar head group.

15. Carrier and targeting system according to any one of the preceding claims, **characterized in that** the phospholipids are selected from the group comprising phosphatidylcholine (PC), phosphatidylethanolamine (PE), phosphatidic acid (PA), Phosphatidyl-inositol (PI), and sphingomyelin (SM).

16. Carrier and targeting system according to any one of the preceding claims, **characterized in that** the two hydrocarbon chains are between 8-26 carbon atoms in length.

17. Carrier and targeting system according to any one of the preceding claims, comprising additionally of glycolipids such as cerebiosides and gangliosides.

18. Carrier and targeting system according to any one of the preceding claims, **characterized in that** the carrier and targeting system is obtainable by:
m) mixing one or more organosilicon, sugar organosilicon, amino-sugar organosilicon, alkyl, alkoxy or acyloxy organosilicon, peptide organosilicon, amino acid(s) organosilicon, lipids and/or their derivatives, salts and/or the vesicles formed from them, with one or more polymers, such as PEG, one or more salts, such as Zn²⁺, Ca²⁺, Mg²⁺, Na⁺, buffers, with one or more active substances, such as doxorubicin, cis-oxoplatin, oxali-platin, nucleic acids, such as DNA, RNA, siRNA, nucleosides at selected pH, salt concentration and temperature;
n) homogenisation, sonication and/or extrusion of the mixture, followed by
o) separation of the free active substance,
p) sterile filtration of the mixture,
q) lyophilisation,
r) reconstitution to form a siosome and/or a combined siosome-liposome complex concentrate.

19. Method for preparing the carrier and targeting system according to any one of the preceding claims, **characterized by**
s) mixing one or more organosilicon, sugar organosilicon, amino-sugar organosilicon, alkyl, alkoxy or acyloxy organosilicon, peptide organosilicon, amino acid(s) organosilicon, lipids and/or their derivatives, salts and/or the vesicles formed from them, with one or more polymers, such as PEG, one or more salts, such as Zn²⁺, Ca²⁺, Mg²⁺, Na⁺, buffers, with one or more active substances, such as doxorubicin, cis-oxoplatin, oxali-platin, nucleic acids, such as DNA, RNA, siRNA, nucleosides at selected pH, salt concentration and temperature;
t) homogenisation, sonication and/or extrusion of the mixture, followed by
u) separation of the free active substance,
v) sterile filtration of the mixture,
w) lyophilisation,
x) reconstitution to form a siosome and/or a combined siosome-liposome complex concentrate.

20. Method for preparing the carrier and targeting system according to claim 19, **characterized in that** the active substance, preferably nucleic acids, DNA, RNA and/or siRNA, can be loaded with cations prior to preparation of the siosome carrier systems.

21. Method for preparing the carrier and targeting system according to claims 19 and 20, **characterized in that** the cationic silanes and/or siosomes can be loaded with anionic salts.

22. Method for preparing the carrier and targeting system according to any one of claims 19 to 21, **characterized in that** the anionic silanes and/or siosomes can be loaded with cationic salts.

23. Method for preparing the carrier and targeting system according to any one of claims 19 to 22, **characterized in that** the mixing is carried out in a buffer solution that comprises TRIS, HEPES; MOPS, MES and/or other commonly used biological buffers.

24. Method for preparing the carrier and targeting system according to any one of claims 19 to 23, **characterized in that** the method includes one or more additives selected from the group comprising of detergent solution, adjuvant solution, salt solution, PEG-conjugates and/or cryoprotectants.

25. A method for administering the carrier and targeting system according to any one of claims 1 to 18, **characterized in that** the carrier system, siosome- and/or siosome-liposome-complex is administered via oral, rectal, vaginal, topical, nasal, intradermal, or parenteral administration.

26. A method for administering the carrier and targeting system according to the preceding claim, whereby the parenteral administration method is selected from the group comprising subcutaneous, intravenous, intramuscular and infusion.

27. A method for treating solid and haematological malignancies in animals and humans comprising the administration of the carrier and targeting system according to any one of claims 1 to 18.

28. Method for increasing intracellular concentration of a siosome-entrapped active substance, preferably doxorubicin, cis-platin and/or siRNA, **characterized by** administering the carrier and targeting system according to any one of claims 1 to 18 to a patient or a target cell.

29. Method according to the previous claim, whereby the intracellular cytotoxicity of a tumor cell or other target cell is increased by administration of the carrier and targeting system according to any one of claims 1 to 18.

30. Method for increasing intracellular concentration of a siosome-entrapped active substance according to the preceding claim, whereby the intracellular concentration of the active substance is increased by specific binding of a ligand to a target cell and/or internalisation of the siosome- and/or siosome-liposome-complex by the target cell.

31. Carrier and targeting system according to any one of claims 1 to 18 for use as a medicament.

32. Pharmaceutical agent comprising the carrier and targeting system according to any one of claims 1 to 18 and a pharmaceutically acceptable carrier.

33. Method of transfecting a cell with an anti-cancer active substance or therapeutic agent such as doxorubicin, cis-platin, carbo-platin, oxali-platin, bleomycin, a nucleic acid, such as DNA, RNA, siRNA, an anti-viral active substance and/or an anti-bacterial agent, using as the transfection vector a carrier and targeting system according to claims 1 to 18 comprising at least one sugar organosilicon compound and/or derivatives thereof according to claims 3 to 6, preferably in addition to PEG and/or a cryoprotectant.
